# EUROPEAN PATENT SPECIFICATION

(11) **EP 4 031 003 B1**
(45) Date of publication and mention of the grant of the patent: **26.08.2026**
(21) Application number: 20785842.4
(22) Date of filing: 21.09.2020
(51) Int. Cl.: A61B 5/113, A61B 5/053, A61B 5/00

(54) **SYSTEM AND METHOD FOR DETERMINING SLEEP STAGES BASED ON NON-CARDIAC BODY SIGNALS**
SYSTEM UND VERFAHREN ZUR BESTIMMUNG VON SCHLAFPHASEN BASIEREND AUF NICHTKARDIALEN KÖRPERSIGNALEN
SYSTÈME ET PROCÉDÉ POUR DÉTERMINER DES STADES DE SOMMEIL SUR LA BASE DE SIGNAUX CORPORELS NON CARDIAQUES

(30) Priority: 20.09.2019 US 201962903478 P; 20.09.2019 US 201962903493 P
(43) Date of publication of application: 27.07.2022
(73) Proprietor: Nox Medical ehf., 105 Reykjavik (IS)
(72) Inventor: HÖSKULDSSON, Sveinbjörn, 105 Reykjavik (IS); ÁGÚSTSSON, Jón Skírnir, 105 Reykjavik (IS); FINNSSON, Eysteinn, 105 Reykjavik (IS)
(74) Representative: Inspicos P/S
(86) International application number: PCT/IB2020/058793
(87) International publication number: WO 2021/053645

(56) References cited:
- US-A1- 2015 230 750
- US-A1- 2017 196 500
- US-A1- 2019 000 375
- US-A1- 2019 150 787
- MEHDI SHOKOUEINEJAD ET AL: "Sleep apnea: a review of diagnostic sensors, algorithms, and therapies", PHYSIOLOGICAL MEASUREMENT, INSTITUTE OF PHYSICS PUBLISHING, BRISTOL, GB, vol. 38, no. 9, 18 August 2017 (2017-08-18), XP020319268, ISSN: 0967-3334, [retrieved on 20170818], DOI: 10.1088/1361-6579/AA6EC6
- YULITA INTAN NURMA ET AL: "Bi-directional Long Short-Term Memory using Quantized data of Deep Belief Networks for Sleep Stage Classification", PROCEDIA COMPUTER SCIENCE, vol. 116, 13 October 2017 (2017-10-13), pages 530 - 538, XP085232345, ISSN: 1877-0509, DOI: 10.1016/J.PROCS.2017.10.042
- YILDIZ SELDA ET AL: "Categorizing Sleep in Older Adults with Wireless Activity Monitors Using LSTM Neural Networks", 2019 41ST ANNUAL INTERNATIONAL CONFERENCE OF THE IEEE ENGINEERING IN MEDICINE AND BIOLOGY SOCIETY (EMBC), IEEE, 23 July 2019 (2019-07-23), pages 3368 - 3372, XP033625356, DOI: 10.1109/EMBC.2019.8857453

## Description

### FIELD OF THE DISCLOSURE

The present disclosure relates to a system, apparatuses, and a method for determining sleep stages of a subject, and particularly for determining sleep stages based on signals obtained from the body of the subject without necessarily being signals obtained from the brain or heart of the subject.

### BACKGROUND

Clinical sleep studies of different types have been developed. Such studies have either focused on measuring or identifying a specific sleep disorder or have been more general for measuring the overall sleep profile along with the signals necessary to confirm or exclude different sleep disorders.

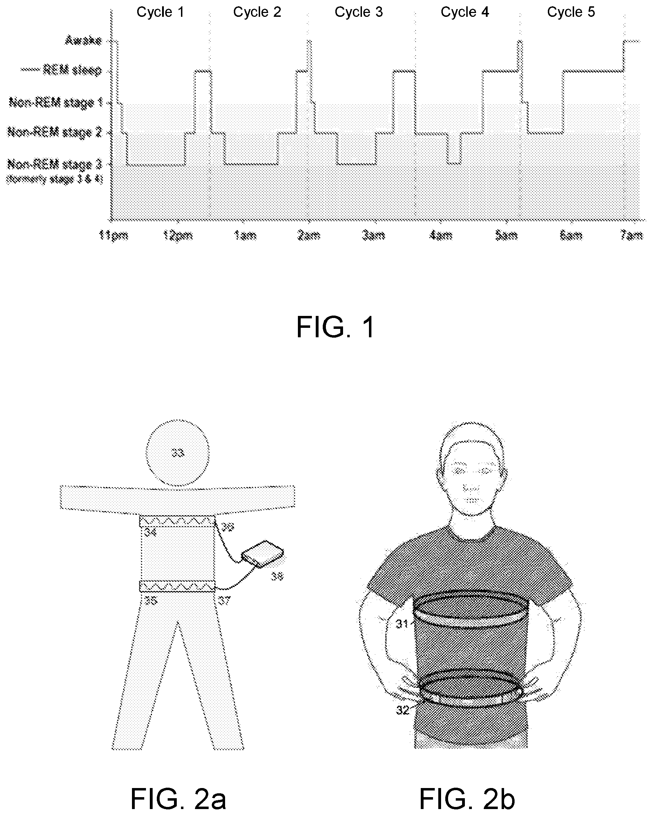

Polysomnography (PSG) is a general sleep study that records miscellaneous physiological signals, including electroencephalography (EEG) signals from the head of a subject for determining sleep stages of the subject. The time people spend in bed can normally be divided into certain periods or stages of Rapid Eye Movement (REM) sleep, Non-rapid eye movement sleep (Non-REM or NREM) sleep, and occasional Wake periods. Standard PSG allows further classification of the NREM periods on different levels of sleep including N1, N2, and N3, with N1 being the shallowest, then N2, and finally N3. The N3 period is often referred to as deep sleep or Slow Wave Sleep due to the slow EEG signals that are characteristic of this period. The sleep stages are often presented in a graph as shown in FIG. 1 with the X axis labeled with the time of day and the Y axis showing 5 values, Wake, REM, N1, N2, N3. A line may then be plotted showing the sleep stage of the subject at different times of the night or sleep study period. Such a graph is called Hypnogram and is the standard presentation of the sleep profile used in PSG studies.

EEG is typically based n electrodes placed on the scalp of the subject. The clinical standards for PSG require that the recording of EEG signals is done with electrodes located on parts of the head typically covered in hair. But a patient or subject generally can't or has difficulty applying the sleep study electrodes on himself, or at least has difficulty applying the sleep study electrodes on himself correctly. Therefore the patient must be assisted by a nurse or technician. For this reason, most PSG studies are done in a clinic, as the patient needs to be prepared for the sleep study around the time he goes to bed.

Another common type of sleep study is Apnea Home Sleep Testing (HST). HST generally only focuses on respiratory parameters and oxygen saturation for diagnosing sleep apnea and sleep disordered breathing. HST does however not require EEG electrodes on the head or sensors that the patient can't place on him himself. Therefore, the most common practice in HST is to hand the HST system to the patient over-the-counter in the clinic or send the HST system by mail to the patient and have the patient handle the hookup or placement of the HST system to himself. This is a highly cost-efficient process for screening for sleep apnea. However, this practice has the drawback that the sleep stages, including time of sleep/wake periods is missing. It is therefore the risk of HST not performed in a clinic that the patient was indeed not sleeping during the whole recording time. But as this may not be known to the technician scoring the data from the HST after the study, there is the risk that this could affect the clinical decision on the severity of the sleep apnea. It would therefore be preferred to have some prediction or determination of the sleep stages of the subject to improve the accuracy of the diagnoses. But as noted above, doing a standard EEG on the patient during the HST would be impractical or impossible in a home-type setting, or too expensive.

It has been found that the heart rate variability is different between NREM and REM or Wake. Additionally, during REM there is very little body movement due to REM paralysis but clearly more during Wake. By using those facts and other known features, a sleep profile can be derived directly from signals obtained from the body of the subject, which may be referred to as "body sleep" to distinguish a sleep study based on signals obtained from the brain of the subject, which may be referred to as "brain sleep," which is typically only measurable using EEG.

A common example of such a "body sleep" study method may be based on cardio signals. Such methods are growing in popularity in the health and consumer device market. For example, many smart watches measure the pulse by the wrist and use it to create features that can provide a simple sleep profile. Some clinical products are similarly using those cardio or cardio-respiratory features to record simple sleep profiles.

Similarly, body movement signals may be obtained and analyzed in a simple "body sleep" study.

A study of "body sleep" based on measured cardio signals or body movements may be sufficient or interesting for health or consumer products, which are often used for entertainment purposes only. But the drawback of using such signals is that such measurements do not work consistently for all people and such measurements become very inaccurate for others. For example, a significant drawback to using cardio signals for estimating sleep patterns is that although this method may work for evaluation of healthy young people with strong hearts, patients with sleep disorders frequently have heart-related issues, such as high blood pressure, arrythmias, and even congestive heart failure. These conditions, along with the drugs used to treat these conditions directly affect the signal features measured in a cardio-based sleep study, such as identifying periods or reduced heart-rate variability during REM.

Also, basing the REM/Wake classification on activity or body movement alone can be very inaccurate, as some patients do not move much while they try to fall asleep. Such periods of inactivity may frequently be misclassified as a REM period. Health and consumer product providers often simply try to overcome this type of misclassification by fitting the patient to the known sleep profiles rather than measuring it directly. For example, one can assume that most heathy people go from Wake into NREM sleep and from NREM to REM before waking up again. As these types of sleep stage profiling is typically meant for entertainment it is not a problem that they might be wrong for patients having sleep disorders that do not fit within the standard profiles. However, for clinical purposes, guessing or assuming the sleep stages simply is not good enough because the irregular sleep profile of a subject is an important part of sleep diagnostics and a guessed profile may lead to an incorrect clinical decision.

It would therefore be of significant benefit if body sleep could be measured without using or without requiring features derived from the heart, or at least features derived solely from the heart. It would also be of benefit if body sleep could be measured based on signals more accurate than simply body movement signals. This would allow the sleep study to be used with improved certainty on cardio patients as well as others and greatly reduce the risk of wrong clinical decisions.

Relevant technology may be seen in: US2019/150787, US2017/196500, US2019/000375 and Mehdi Shokoueinejad et al "Sleep apnea: a review of diagnostic sensors, algorithms, and therapies", Physiological measurement, Institute of physics publishing,

Bristol, GB, Vol. 38, No. 9, 18-8-2017. US2015/230750 discloses a sleep stage monitor that may access sensor data signals related to bodily movement and/or respiration movements. At least a portion of the detected signals may be analyzed to calculate respiration variability. The respiration variability may include one or more of variability of respiration rate and variability of respiration amplitude. A processor may then determine a sleep stage based on one or more of respiration variability and bodily movement, such as with a combination of both. The determination of sleep stages may distinguish between deep sleep and other stages of sleep, or may differentiate between deep sleep, light sleep and REM sleep. The bodily movement and respiration movement signals may be derived from one or more sensors, such as noninvasive sensor e.g., a non-contact radio-frequency motion sensor or a pressure sensitive mattress) but could also be derived from respiratory inductance plethysmography.

### SUMMARY

A non-invasive method system and a hardware storage device are provided according to claims 1, 8 and 14.

### BRIEF DESCRIPTION OF THE DRAWINGS

FIG. 1 shows a sleep histogram (hypnogram) illustrating the transition between sleep stages during an ideal night of sleep.
FIGS. 2a and 2b illustrate an example of respiratory inductance plethysmograph (RIP) belts.
FIG. 3 shows an exhalation part of a flow signal.
FIG. 4 shows a spectral density of the signal in FIG. 3
FIG. 5 shows an example of the RIPsum signal of breathing during sleep.
FIG. 6 shows identified End/Start points and Midway points in a RIPsum signal with noise present.
FIG. 7 shows an example of how points from a RIPsum signal of breathing during sleep can be grouped.
FIGS. 8a and 8b show the probability of changing a group of points of RIPsum signal of breathing as a function of the amplitude of the group minima and maxima.
FIG. 9 shows a structure of a single gated recurrent unit (GRU) unit.
FIG. 10 shows a diagram of the neural network.
FIG. 11a shows a distribution of sleep stages for a cross-validation set.
FIG. 11b shows a distribution of sleep stages for ta test set.
FIG. 11c shows Apnea-Hypopnea Index (AHI) versus F1-score (to the right) and Cohen's Kappa (to the left) for combined datasets.
FIG. 11d shows an average F1-score for recordings in different AHI categories on the combined datasets.
FIG. 11e shows BMI versus average F1-score for recordings in the First Dataset
FIG. 11f shows a distribution of F1-scores for females (left) and for males (right) for recordings in the First Dataset.

### DETAILED DESCRIPTION OF VARIOUS EMBODIMENTS

As noted above, it would be preferred to have some prediction, determination, or classification of the sleep stages of a subject to improve the accuracy of a sleep-related diagnoses. But doing a standard electroencephalography (EEG) on the patient during an Apnea Home Sleep Testing (HST) is often impractical or impossible in a home-type setting, or is too expensive.

Additionally, a sleep study including a sleep stage prediction, determination, or classification based on cardio or heart-related signals or body movement signals are often inaccurate.

What would be preferred is that a sleep stage determination be performed in a body sleep study without using or at least without requiring features derived from the heart, such as a body sleep, sleep stage determination based on breathing features without requiring heart-related signals. It would also be preferred that a body sleep, sleep stage determination could be measured on more features than body movement signals. This would allow the sleep study could be performed with improved certainty on cardio patients as well as others and would greatly reduce the risk of wrong clinical decisions.

The inventors of the present disclosure have found that this can be done by using the fact that the sleep stage of the brain affects other body functions, such as breathing, heart function and an interaction between the two. It is therefore possible to use features and characteristics of breathing and heart signals to predict the sleep stage of the patient.

As noted above, it has been found that the heart rate variability is different between NREM and REM or Wake. There is a strong synchrony between respiration and pulse during NREM but low during REM or Wake. Additionally, during REM there is very little body movement, but more during Wake periods. By using these facts, a sleep profile can be derived from the body signals and a body sleep, sleep stage determination can be made that is not based on or does not require cardio or heart-based signals or brain-based signals, such as EEG.

As used herein, a method, sensor, or procedure may be described as non-invasive when no break in the skin is created and there is no contact with the mucosa, or skin break, or internal body cavity beyond a natural body orifice. In the context of sleep studies or determining a sleep stage of a subject, the term invasive may be used to describe a measurement that requires a measurement device, sensor, cannula, or instrument that is placed within the body of the subject, either partially or entirely, or a measurement device, sensor, or instrument placed on the subject in a way that interferes with the sleep or the regular ventilation, inspiration, or expiration of the subject. For example, a measuring of esophageal pressure (Pes), which is considered the gold standard in measuring respiratory effort, requires the placement of a catheter or sensor inside the esophageal and is therefore considered an invasive procedure and is not practical for general respiratory measures. Other known output values can be derived from invasive measurements, such as direct or indirect measure of intra thoracic pressure PIT and/or diaphragm and intercostal muscle EMG. as esophageal pressure (Pes) monitoring, epiglottic pressure monitoring (Pepi), chest wall electromyography (CW-EMG), and diaphragm electromyography (di-EMG). Each of these methods suffers from being invasive.

Non-invasive methods to measure breathing movements and respiratory effort may include the use of respiratory effort bands or belts placed around the respiratory region of a subject. The sensor belt may be capable of measuring either changes in the band stretching or the area of the body encircled by the belt when placed around a subject's body. A first belt may be placed around the thorax and second belt may be placed around the abdomen to capture respiratory movements caused by both the diaphragm and the intercostal-muscles. When sensors measuring only the stretching of the belts are used, the resulting signal is a qualitative measure of the respiratory movement. This type of measurement is used, for example, for measurement of sleep disordered breathing and may distinguish between reduced respiration caused by obstruction in the upper airway (obstructive apnea), where there can be considerable respiratory movement measured, or if it is caused by reduced effort (central apnea), where reduction in flow and reduction in the belt movement occur at the same time.

Unlike the stretch-sensitive respiratory effort belts, areal sensitive respiratory effort belts provide detailed information on the actual form, shape and amplitude of the respiration taking place. If the areal changes of both the thorax and abdomen are known, by using a certain calibration technology, the continuous respiratory volume can be measured from those signals and therefore the respiratory flow can be derived.

The inventors have developed a method for determining body sleep based on breathing and body activity features but excluding or at least not requiring cardio features. For example, the method may be based on using only the signals from one or more respiratory inductance plethysmography (RIP) belts intended for measuring respiratory movements of the thorax and abdomen. FIGS. 2a and 2b illustrate an example of respiratory inductance plethysmograph (RIP) belts. FIG. 2a shows an example of the wave-shaped conductors in the belts, and FIG. 2b shows the cross-sectional area of each belt, which is proportional to the measured inductance.

Respiratory Inductive Plethysmography (RIP) is a method to measure respiratory related areal changes. As shown in FIGS. 2a and 2b, in RIP, stretchable belts 31, 32 may contain a conductor 34, 35 that when put on a subject 33, form a conductive loop that creates an inductance that is directly proportional to the absolute cross sectional area of the body part that is encircled by the loop. When such a belt is placed around the abdomen or thorax, the cross-sectional area is modulated with the respiratory movements and therefore also the inductance of the belt. Conductors 34, 35 may be connected to signal processor 38 by leads 36, 37. Processor 38 may include a memory storage. By measuring the belt inductance, a value is obtained that is modulated directly proportional with the respiratory movements. RIP technology includes therefore an inductance measurement of conductive belts that encircle the thorax and abdomen of a subject. As used herein, a respiratory signal may be obtained bv the respiratory signal being received by a processor directly from the RIP belts, by a processor receiving a pre-processed respiratory signal that had originally been obtained from the RIP belts, or a respiratory signal may be obtained by a processor by the processor receiving a respiratory signal that was previously obtained from a subject and stored on a memory storage, either in a raw unprocessed form or in a pre-processed form, and subsequently obtained or received by the processor from the memory storage. The memory storage may be a separate device from the processor, may be hardwired to the processor, or the stored respiratory signal may be transmitted to the processor, for example, over a network or another communications connection (either hardwired, wireless, or a combination of hardwired or wireless) to a computer system that includes the processor.

In another embodiment, conductors may be connected to a transmission unit that transmits respiratory signals, for example raw unprocessed respiratory signals, or semi-processed signals, from conductors to processing unit. Respiratory signals or respiratory signal data may be transmitted to the processor by hardwire, wireless, or by other means of signal transmission.

Resonance circuitry may be used for measuring the inductance and inductance change of the belt. In a resonance circuit, an inductance L and capacitance C can be connected together in parallel. With a fully charged capacitor C connected to the inductance L, the signal measured over the circuitry would swing in a damped harmonic oscillation with the following frequency: $f = \frac{1}{2 π \sqrt{LC}}$ until the energy of the capacitor is fully lost in the circuit's electrical resistance. By adding to the circuit an inverting amplifier, the oscillation can however be maintained at a frequency close to the resonance frequency. With a known capacitance C, the inductance L can be calculated by measuring the frequency f and thereby an estimation of the cross-sectional area can be derived.

The method for determining body sleep based on breathing and body activity features but excluding or at least not requiring cardio features may also include using a signal from an activity sensor. The method uses a new feature based on the difference between the two RIP signals as an addition to the Wake/REM classification and greatly increases the accuracy of that problematic task. Similarly, NREM stages may be accurately distinguished from the Wake and REM periods. This sleep stage classifying method and system therefore delivers the WAKE/NREM/REM profile of a subject, while not necessarily trying to further classify the NREM into N1, N2, and N3. This is however sufficient to significantly increase the information on the sleep of a patient undergoing HST, for example, and corrects the sleep time and allows the sleep physician to conclude if sleep disordered breathing is only happening during REM. Such a conclusion could lead to a different treatment option.

As described below, the method and system may be based on using a Nox HST recorder to record RIP and body activity signals during the night and then subsequently uploading recorded data signals to a computer after the study is completed. Of course, other HST recording devices may be used. Software may be used to derive multiple respiratory and activity parameters from those signals, such as respiratory rate, delay between the signals, stability of the respiration and ratio of amplitude between the two belts.

When the parameters have been derived, they may then be fed into a computing system. For example, in a first embodiment the parameters are fed into an artificial neural network computing system that has been trained to predict the three sleep stages, Wake, REM and NREM, which may be used to plot a simplified hypnogram for the night. The classifier computing system might be different than artificial neural network. For example, in another embodiment a support vector machine (SVM) method could be used, clustering methods could be used, and other classification methods exist which could be used to classify epochs of similar characteristics into one of several groups. In the first embodiment of the method, an artificial neural network was used. This method can be used on a standard HST, does not add any burden to the patient or subject, and may be provided in a fully automated way by the physician.

### 1 - Introduction

In this disclosure, the design of a new sleep stage classifier for polygraphy (PG) recordings is provided. The rules of the American Academy of Sleep Medicine (AASM) specify that when scoring sleep stages for PSG recordings, each recording should be split into 30-second long epochs and each epoch should be labeled Wake, REM, N1, N2 or N3 by looking at the EEG. However, in a PG sleep study, only the respiration signals are recorded and there exist no guidelines for scoring sleep stages in such studies. A new automatic sleep stage classifier for PG recordings is provided herein by a system or method which relies only on RIP belts, or on RIP belts and a body movement sensor, such as an accelerometer. The task has been reduced to a three-stage classification with the stages being Wake, REM and NREM. Or in another embodiment, the sleep stages may be reduced to Wake, REM, N1, N2, N3. However, to simplify the method, the sleep stages N1, N2, and N3 may be reduced to NREM.

This disclosure describes the technical aspects of the PG+ sleep stage classifier. First, the dataset used for developing and validating the method is described. Next, the feature extraction method is discussed, including a description of each feature. The model used for the classification task is then discussed, as well as the training of the model. Finally, the results are presented, and a discussion of things tried is included.

### 2 - Datasets

Two datasets were used for the development and validation of the classifier described herein. The first dataset includes 179 PSG recordings that were recorded using the NOX A1 system (hereinafter referred to as the "First Dataset").

The second dataset includes 186 recordings using the NOX A1 system (hereinafter referred to as the "Second Dataset").

The full dataset includes 349 recordings of which 186 had been manually scored.

For each dataset, 85% is used for training and validation and the remaining 15% has been kept as a hidden test set.

### 3 - Feature Extraction

The classification task is a two-part problem with the first step in the process being the extraction of features from the raw recordings. In an embodiment, a feature extractor was written in Python 3.5.5 to perform this task. The extractor may rely on NumPy and/or SciPy. The output of the feature extractor is a comma-separated values (CSV) file where the rows represent each epoch and the columns contains the features.

In a first embodiment, the signals used are those derived from the abdomen and thorax RIP belts. These include the Abdomen Volume, Thorax Volume, RIPSum, RIPFlow, Phase, and RespRate signals. Additionally, an activity signal from an accelerometer was used. All the features were calculated over a 30s epoch. The total number of features used in this version are 61. However, other numbers of total features used may be more or less. This chapter has been divided into sections corresponding to the feature extraction files in the project.

As used herein, Abdomen Volume and Thorax Volume are the RIP signals recorded during the sleep study. The signals may be recorded using the respiratory inductance plethysmography (RIP) bands placed around or on the thorax and abdomen of the subject under study. The RIP signals represent volume in the abdomen and thorax during breathing.

RIPSum is a signal created by adding the samples of Abdomen Volume and Thorax Volume signals. The RIPSum signal is a time series signal of the same number of samples and duration in time as the Abdomen Volume and Thorax Volume signals.

RIPFlow is the time derivative of the RIPSum signal. The RIPSum signal represents volume and the time derivative represents changes in volume which is flow.

Phase is a signal represents the apparent time delay between the recorded Abdomen and Thoracic volume signals. During normal unobstructed breathing the Abdomen and Thorax move together out and in during inhalation and exhalation. When the upper airway becomes partially obstructed the Abdomen and Thorax start to move out of phase, where either the Abdomen or the Thorax will start expanding while pulling the other back. During complete obstruction of the upper airway the Abdomen and Thorax will start moving completely out of phase, whereas one moves out the other one is pulled inwards. In this case the Phase is 180 degrees, measuring the phase difference between the two signals.

RespRate represents the respiratory rate of the subject under study. The respiratory rate is a measure of the number of breaths per minute and is derived from the Abdomen Volume and Thorax Volume signals.

The feature extractor and the features extracted by the feature extractor are explained herein below. The main points in the description of feature extractor and the features extracted by the feature extractor are:
- It works on the recorded signals of Abdomen RIP, Thorax RIP, and accelerometers.
- It works on signals derived from the above-mentioned recorded signals. These are the RIPSum, RIPFlow, Phase, RespRate, and Activity.
- It splits the signals into 30 second epochs which are used to calculate the features.
- Is may be implemented in Python using NumPy and SciPy. This is not an essential feature of the method, just how it was done in in an embodiment.
- It outputs results in a CSV file. This is not an essential feature of the method, just how it was done in an embodiment.

### 3.1 - Respiratory Rate

The respiration features are calculated from the RIPSum, RIPFlow and RespRate signals. The features calculated were designed to give information about changes in the respiratory rate with various methods.

### 3.1.1 First Harmonic-to-DC Ratio

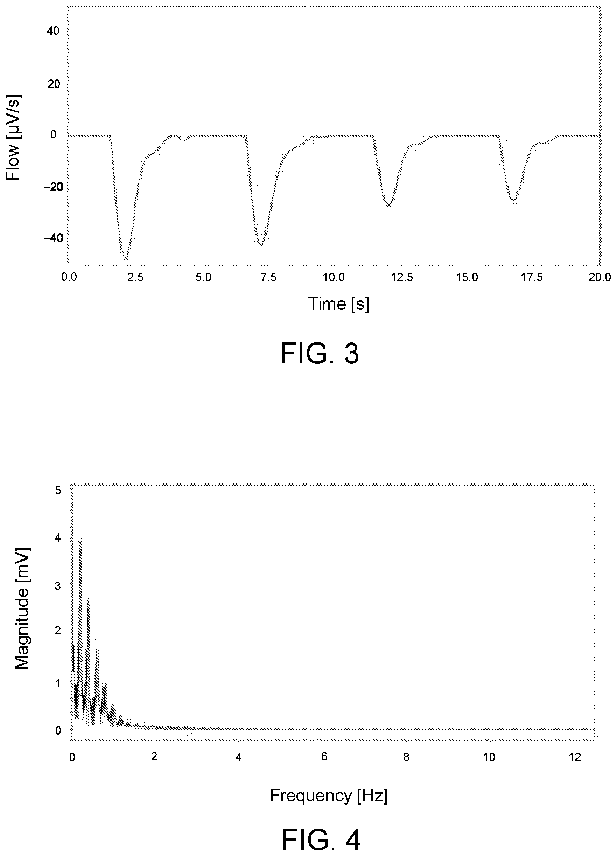

The first harmonic and DC ratio is used to estimate respiratory rate variability. The first harmonic and the DC component are found in the frequency spectrum of a flow signal. For this classifier the RIPFlow was used but some preprocessing required. Such preprocessing included before taking the Fourier transform of the signal, all positive values are made 0, which results in the signal being more periodic as the exhalation is more regular. This can be seen in FIG. 3.

The fast Fourier transform is applied on the resulting signal and the DC component and the first harmonic peak are located. The DC component is defined as the magnitude at 0 Hz and the first harmonic peak is the largest peak of the frequency spectrum after the DC ratio, as can be seen in FIG. 4.

The respiratory rate variability with this method may be defined as: $RRv = \left(100-\frac{{H}_{1}}{DC}\right) %$

Where H₁ is the magnitude of the first harmonic peak and DC is the magnitude of the DC component. It has been showed that the RRv is larger in wake and that this size gets smaller as the sleep gets deeper but is larger again in REM sleep. The feature implemented in the final version is just the first harmonic to DC ratio but not the RRv value, since after normalization these values would still be the same.

### 3.1.2 - Respiratory Rate

There may be 4 features that are extracted from the respiratory rate. These features are calculated using mean, standard deviation and difference between epochs. The RespRate signal is used for these calculations. The mean and standard deviation of the respiratory rate is calculated for each epoch. The root means square successive difference (RMSSD) is calculated with $RMSSD = \sqrt{\frac{1}{N - 1} {\sum }_{i = 1}^{N - 1} {\left({RR}_{i}-{RR}_{i - 1}\right)}^{2}}$

The difference mean ratio is then calculated as the ratio of the mean respiratory rate of the current epoch and the previous epoch.

### 3.2 - Breath-by-breath

The breath-by-breath features are based on features which are calculated for each breath. The final features are then calculated by taking the mean, median or standard deviation of the breath features for each epoch. The breaths may be located by running a breath-by-breath algorithm on the RIPsum signal of the whole recording to identify all the breaths. The breaths may then be divided between the 30s epochs, with breaths that overlap two epochs being placed in the epoch that contains the end of the exhalation of the breath. The signals used for the feature calculations are the RIPsum, RIPflow, Abdomen Volume and Thorax Volume.

In a second embodiment, the breath-by-breath algorithm may be based on a start of inhalation being marked as the start of a breath and the end of exhalation being marked as the end of a breath. By adding the correctly calibrated abdomen and thorax RIP signal, calculating a time derivative of the resulting calibrated RIP volume signal results in a flow signal representing breathing airflow. The start of inhalation can be determined by finding points in time where the flow signal crosses a zero value from having negative values to having positive values. The end of exhalation can be determined by finding points in time where the flow signal crosses a zero value from having negative values to having positive values.

What is meant by this is that when the RIP flow signal has a positive value air is flowing into the body, inhalation, and when the RIP flow signal has a negative value air if slowing out of the body, exhalation.

This may not be the most sophisticated method of detecting inhalation and exhalation. But at the same time, for all practical purposes it is not bad and widely used. It may be noted that high frequency noise in the signal might cause the signal to oscillate, causing multiple zero crossings in periods where the flow rate is low. But to this it can be answered that normal breathing frequency is around 0.3 Hz, so low pass filtering the signal at a frequency around 1-3 Hz can be applied to remove high frequency noise.

Detecting individual breaths in a sleep recording can be done by using the abdomen RIP signal, the thorax RIP signal, or their sum (RIPsum). Breath onset is defined as the moment when the lungs start filling with air from their functional residual capacity (FRC) causing the chest and abdomen to move and their combined movement corresponding to the increase in volume of the lungs. Functional Residual Capacity is the volume of air present in the lungs at the end of passive expiration and when the chest and abdomen are in a neutral position.

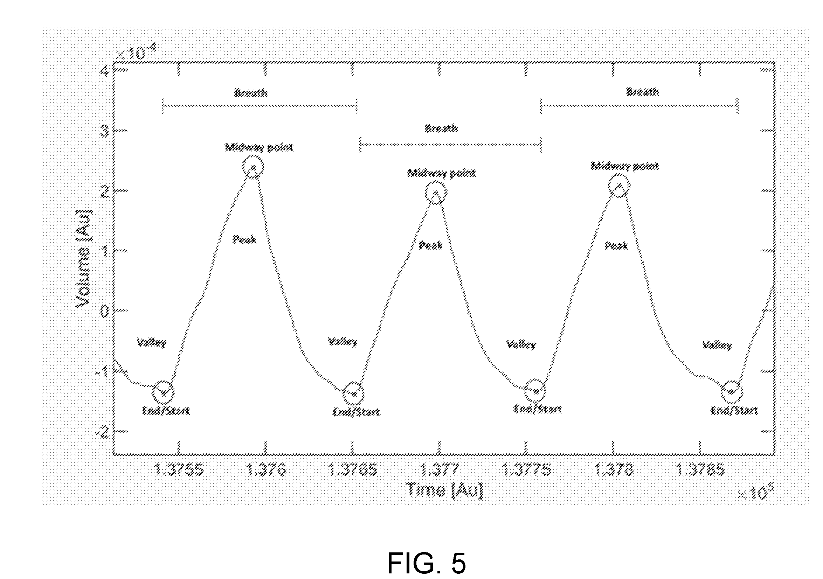

FIG. 5 shows an example of the RIPsum signal of breathing during sleep. FIG. 5 shows how the RIPsum starts at a lower bound, End/Start, and rises to an upper bound, Midway point, before it falls back down. The rise of the signal indicates the breath onset. A naive or simple method of detecting the breath onset is to look for points where the derivative of the signal changes sign from negative to positive, or when the derivative crosses the zero value from negative to positive and label them as End/Start. Points where the sign of the derivative changes from positive to negative are the Midway points. However, this naive or simple method suffers from misidentification of End/Start points and Midway points in the presence of noise.

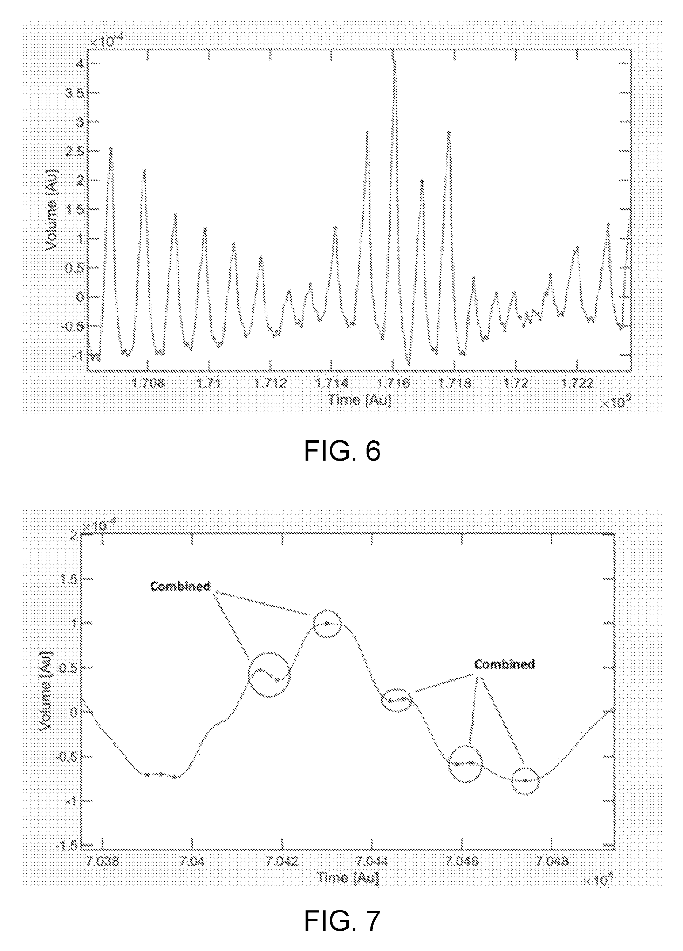

FIG. 6 shows identified End/Start points and Midway points in a RIPsum signal with noise present.

In the presence of noise, too many points can be identified as End/Start points or Midway points. To mitigate this one can low-pass filter the signal at a frequency high enough to capture the breathing movement and low enough to remove most noise. A cutoff frequency of, for example, 3 Hz could be used, as it is around ten times higher than the breathing frequency. A second mitigation strategy is to investigate the End/Start points and Midway points and identify points which represent noise. One strategy to combine points is to define a threshold value in the signal amplitude which needs to be passed before defining a new End/Start point or a new Midway point.

FIG. 7 shows an example of how points can be grouped. During a period where the signal is increasing, a local maxima is identified and registered as a possible Midway point. Following this point a possible End/Start point is identified. However, since the amplitude difference between the possible Midway point and End/Start point does not exceed the threshold value the points are combined, as identified by the circle. Now the signal is considered to still be rising and the following local maxima is identified as the possible Midway point. The difference in amplitude from this possible Midway point to the following local minima exceeds the threshold and this point is determined to be the true Midway point and combined with the first two points. When looking for the next End/Start point the following local minima is a possible candidate. However, since the local maxima following it does not exceed the threshold these points are combined, and the next local minima investigated. As the process continues the lowest local minima is determined as the End/Start Point.

To further improve the breath detection, time information can be incorporated. By assigning a probability of combining points based on their amplitude difference and distance in time the algorithm described above can be refined.

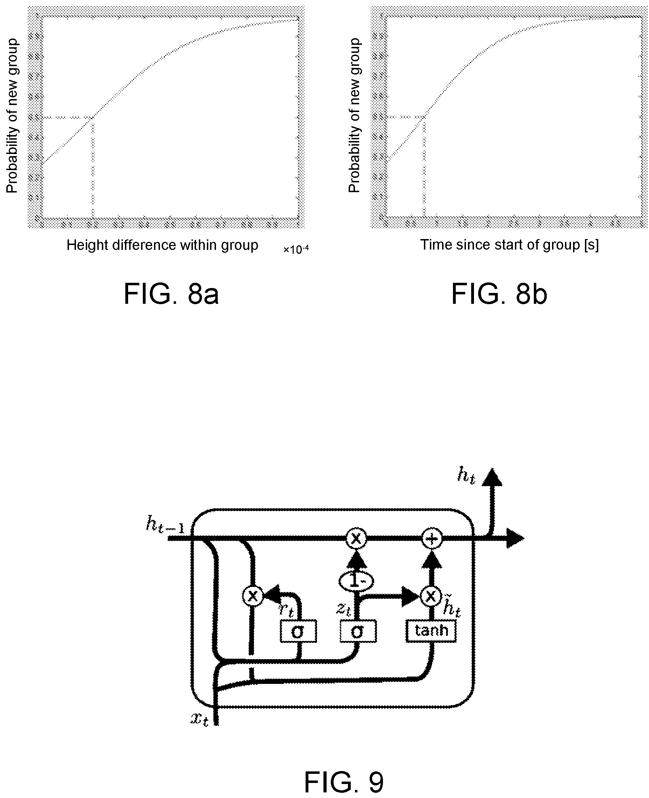

FIGS. 8a and 8b show the probability of changing a group as a function of the amplitude of the group minima and maxima, and as a function of the time passed from the first to the last point in the group. By multiplying the two probabilities the algorithm reduces the likelihood of a period of no breathing, such as apnea or central apnea, being counted as a breath.

### 3.2.1 - Correlation

The correlation feature is based on the similarity of adjacent breaths. To evaluate their similarity the cross-correlation is used with the coefficient scaling method. The coefficient scaling method normalizes the input signals, so their auto-correlation is 1 at the zero lag. The cross-correlation is calculated for each adjacent pair of breaths and the correlation of the breaths is found as the maximum value of the cross-correlation. The last breath of the previous epoch is included for the correlation calculation of the first breath of the current epoch. The mean and standard deviation are then calculated over each epoch. The RIPSum signal is used for these calculations.

### 3.2.2 - Amplitude and Breath Length

The breath length for each breath is calculated along with the inhalation and exhalation durations. This may be done using the start, end and peak values returned by the breath finder. For each epoch then the mean and standard deviation of these lengths was calculated. The median peak amplitude of the RIPsum signal is also calculated for each breath over an epoch.

The median volume and flow of the inhalation, exhalation and the whole breath are calculated for each breath and then the median of all breaths within each epoch is calculated. Along with that, the median of the amplitude of each breath is calculated and the median value of all breaths within each epoch is calculated. This results in 6 features.

### 3.2.3 - Zero Flow

The zero-flow ratio is calculated by locating the exhalation start of each breath. The difference of the amplitude at exhalation and inhalation start is calculated for the abdomen and thorax volume signals and the ratio of the abdomen and thorax values are calculated for each breath. The mean and standard deviation of these values are then calculated for each epoch.

### 3.3 - Activity

For the activity features the standard deviation over 30 second epoch is calculated and the maximum and minimum difference over 30 second epochs is as well calculated. The activity features may be calculated using the activity signal. The activity signal is calculated by $\frac{d}{dt} \sqrt{{x}^{2} + {y}^{2}}$ Where x and y are the x and y component, moving in the horizontal plane, of the 3D accelerometer signal.

### 3.4 - Old Respiratory Features

These features are based on Matlab code from Research Rechtscaffen. These features use the RIPsum, RIPflow, RIPPhase Thorax Volume and Abdomen Volume signals. Some of the features use the Abdomen and Thorax Flow signals which were calculated by numerical differentiation from the volume signals. The features that use the breath-by-breath algorithm use it in the same way as the breath features in the chapter 3.2.

### 3.4.1 - RIP Phase

The mean and standard deviation of the RIPphase signal are calculated over each 30s epoch.

### 3.4.2 - Skewness

Skewness is a measurement on the asymmetry in a statistical distribution. This can be used to look at if the breaths are more skewed to the inhalation part or the exhalation part. It can be seen that the breathing patterns change or how the breathing rhythm changes. The skewness is the 3rd standardized moment and is defined as ${γ}_{1} = \frac{{μ}_{3}}{{μ}_{2}^{3 / 2}}$

To calculate the skewness of the breath it is interpreted as a histogram. The signal is digitized somehow, for example, by scaling it between 0-100 (a higher number can be used for more precision) and converted to integers. The skewness may be calculated by at least two ways at this point. The first method is to construct a signal that has the given histogram and then use built-in skewness functions. The second method is based on calculating the skewness directly by calculating the third moment and the standard deviation using the histogram as weights. First, a signal is made x = (1, 2, ..., n-1, n) where n is the length of the original signal. Then the weighted average is calculated with $μ = \frac{1}{N} {\sum }_{i = 1}^{n} \left({x}_{i}∗{k}_{i}\right)$ where k is the original signal $N = {\sum }_{i = 1}^{n} {k}_{i}$ is the weighted length of x. The weighted third moment is then calculated with ${μ}_{3} = \frac{1}{N} {\sum }_{i = 1}^{N} {k}_{i} {\left({x}_{i}-μ\right)}^{3}$ and the weighted standard deviation with $σ = \sqrt{\frac{1}{N} {\sum }_{i = 1}^{N} {k}_{i} {\left({x}_{i}-μ\right)}^{2}}$

The skewness is then calculated with equation 3.4

This may be done for each breath and the mean and standard deviation of the breaths within one 30 second epoch are calculated. The skewness is calculated for the abdomen, thorax and RIP volume traces. The RIPSum may be used to obtain locations of each breath.

### 3.4.3 - Max Flow In vs Out

The ratio of the maximum flow in inhalation and exhalation may be found by first subtracting the mean from the flow signal and then dividing the maximum of the signal with the absolute of the minimum of the signal. The mean of this ratio may be calculated over 30 second epochs. This ratio is both calculated for the abdomen flow and the thorax flow signals.

### 3.4.4 - Time Constant

The time constant of inhalation and exhalation may also be used as features for the classifier. The time constant *τ* is defined as the time it takes the signal to reach half its maximum value. This is done by first subtracting the minimum value from the whole signal so that the minimum value of the signal is at zero. Half the max value is then subtracted so that the half-way point is at 0 and max(f) = -min(f). Taking the absolute value of the signal then results in a v-shaped signal and the halfway point is then found by finding the lowest point of the signal. The formula is as follows: $τ = arg {min}_{t} \left(abs\left(f\left(t\right)-\frac{{max}_{f} f \left(t\right) - {min}_{f} f \left(t\right)}{2}\right)\right)$

The time constant may then be calculated for inhalation and exhalation of each breath and averaged over the epoch. This is calculated on each volume signal and their corresponding flow signal. In total this results in 12 features, but of course more or less features may be used.

### 3.4.5 - Breath Length

Breath length features may also be included, which may be calculated for all volume signals and their corresponding flow signals. First, the peak of the breath is found as the maximum value of the breath. The start of the breath is then found as the minimum value on the left side of the breath and the end as the minimum value on the right side. The inhale, exhale and total length of each breath is then calculated. The breaths are fetched with the breath-by-breath algorithm on the RIPSum signal. This results in total of 18 features, but of course more or less features may be used.

### 4 - Pre-Processing

The CSV files with the features for each recording may be loaded up in Python. Before any training or classification is started, some pre-processing is required or preferable. The pre-processing may involve normalizing the features for each recording, to make the features independent of the subject in question. For example, if we have subject A with heart rate of 80±5 bpm and subject B with heart rate 100±10, they cannot be compared directly. To make them comparable we use the z-norm which may be defined as $\overline{Z} = \frac{\overline{x} - μ}{σ}$

Where *x̅* is a feature vector, *µ* is the mean of the feature vector, and *σ* is the standard deviation of the vector. By using the z-norm, each feature takes the value of 0±1 and they are therefore independent of subjects and are comparable between sleep stages.

The pre-processing also involves converting the labels from strings ('sleep-wake', 'sleep-rem', 'sleep-n1', sleep-n2', 'sleep-n3') to numbers (0, 1, 2, 2, 2). The five given sleep stages may thus be mapped to three stages: 0 - wake, 1 - REM, 2 - NREM. The labels are then one-hot-encoded as required by the neural network architecture. To explain further, if an epoch originally has the label 'sleep-n2', it will first be assigned the number 2, and then after one-hot encoding, the label is represented as [0, 0, 1].

### 5 - Classifier

The use of neural networks was explored for the classification task, as neural networks are well suited to learn from large and complex datasets. The use of gated recurrent units (GRU) was explored as gating mechanism to make the classification more time and structure dependent. GRU is a special type of recurrent layer that takes a sequence of data as an input instead of a single instance. GRU provides the network to see the ability to capture the time variance of the data, that is it can see more than just the exact moment it is trying to classify. The structure of a GRU unit can be seen in FIG. 9.

The implementation and training of the neural network was performed in Python, using the Keras machine learning library, with TensorFlow backend. TensorBoard was used to visualize and follow the progress of the training in real-time.

### 5.1 - The architecture of the final classifier

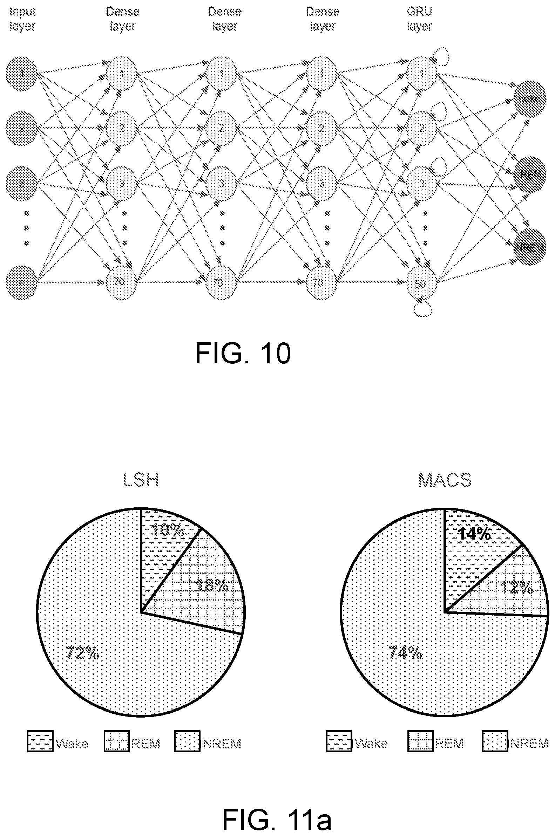

After experimenting with different neural network architectures and tuning hyperparameters (see chapter 7.2.2), a robust classifier was converged on. In this embodiment, the final classifier is a neural network, having three dense layers (each with 70 nodes), followed by a recurrent layer with 50 GRU blocks. The output layer of the network has of 3 nodes, representing for each timestep the class probabilities that the given 30 sec. input window belongs to the sleep stages wake, REM and NREM, respectively. A diagram of an example network can be seen in FIG. 10 where n is the number of features fed to the network.

As our final classifier is indeed a recurrent neural network, with preceding dense layers, the feature matrix must be reshaped before training to the shape *nrₛₐₘₚₗₑₛ* * *nrₜᵢₘₑₛₜₑₚₛ * nr_{features}.* After tuning the number of timesteps, it was found that 25 timesteps give best results. Thus, for each recording a moving window of 25 epochs was taken and for each window a matrix of size 25 ** nr_{features}* was created. After tuning the position of the label, it was found that placing the label at epoch 23, gave a preferable result. Thus, the first 22 epochs represent the past, and the last 2 epochs represent the future.

To explain further, if we have 150 samples of the feature set with 61 features so the data we may have the shape 150x61. We then apply the moving window of size 25 and that results in a data matrix of the shape (150-25)x25x61 = 125x25x61.

Also, because of this design, the first 22 epochs and last 2 epochs of each recording are not scored with the recurrent neural network. Thus, a simple dense neural network is trained to predict the first and last epochs. The dense neural network has the same architecture and same training parameters as the final recurrent neural network, except the forth hidden layer is a 70-node dense layer, instead of a 50-node recurrent layer.

The architecture and hyperparameters for the recurrent neural network can be seen in Table 5.1.

**Table 5.1 The parameters of the Recurrent Neural Network**

| Model Parameters | |
|---|---|
| Input layer shape | (nr_of_samples, 25, nr_of_features) |
| Number of hidden layers | 4 |
| Type of hidden layers | [Dense, Dense, Dense, GRU] |
| Number of hidden units | [70, 70, 70, 50] |
| Dropout for hidden layers | [0.22, 0.22, 0.22, 0.22] |
| Activation Functions for hidden layers | [ReLU, ReLU, ReLU, ReLU] |
| Output layer (number of units, activation function) | 3 nodes, Softmax |
| Regularizer | 0 |
| Weights | Wake: 1, REM: 0.7, NREM: 0.6 |

### 5.2 - Training of the classifier

A combination of the First Dataset and the Second Dataset (described in chapter 2) was used to train the model. The model was trained on 85% of the 365 recordings available at the time of the time of implementation, both via cross-validation, and on the entire 310 recordings, as described in chapter 6.1. The training parameters used can be found in Table 5.2.

**Table 5.2 The parameters of the Neural Network**

| Training Parameters | |
|---|---|
| Optimizer | Adam |
| Loss | Categorical Cross entropy |
| Batch Size | 120 |
| Epochs | 50 |
| Learning Rate | 0.0001 |
| Patience for reducing learning rate | 2 |
| Factor of reducing learning rate | 0.4 |
| Min delta for reducing learning rate | 0.0001 |
| Patience for early stopping | 5 |
| Min delta for early stopping | 0 |
| Beta 1 | 0.9 |
| Beta 2 | 0.999 |
| Epsilon | 1e-8 |
| Decay | 0.0 |

### 6 - Results

In this chapter the validation setup will be explained, and the characteristics of the validation set described. The results of the validation of the PG+ sleep stage classifier will further be reported and discussed.

### 6.1 - Validation dataset and setup

A five-fold cross-validation was performed, both to tune hyper-parameters, and for final validation. Folds were split across subjects, that is the data from a subject can either be part of the training or validation set, but not both. To create the folds, 85% of our subjects was partitioned into 5 groups of approximately equal size, and each group constituted the validation data for one of the 5 folds, while the remaining 4 groups constituted the corresponding training set. A final test set compromising 15% of the dataset was kept aside and not used for the cross-validation. The validation was performed both on the combined datasets, as well as on only the First Dataset and only on the Second Dataset. Unless otherwise states, results shown in this report are based on the validation on only the First Dataset (but trained on the combined dataset), as the First Dataset includes clinical PSG recordings.

FIG. 11a shows the distribution of sleep stages amongst the training sets for the First Dataset and the Second Dataset, according to the manual scorings. As suspected, NREM is the most prevalent sleep stage in both datasets and wake is the least common sleep stage in the First Dataset, but interestingly in the Second Dataset wake is more common than REM.

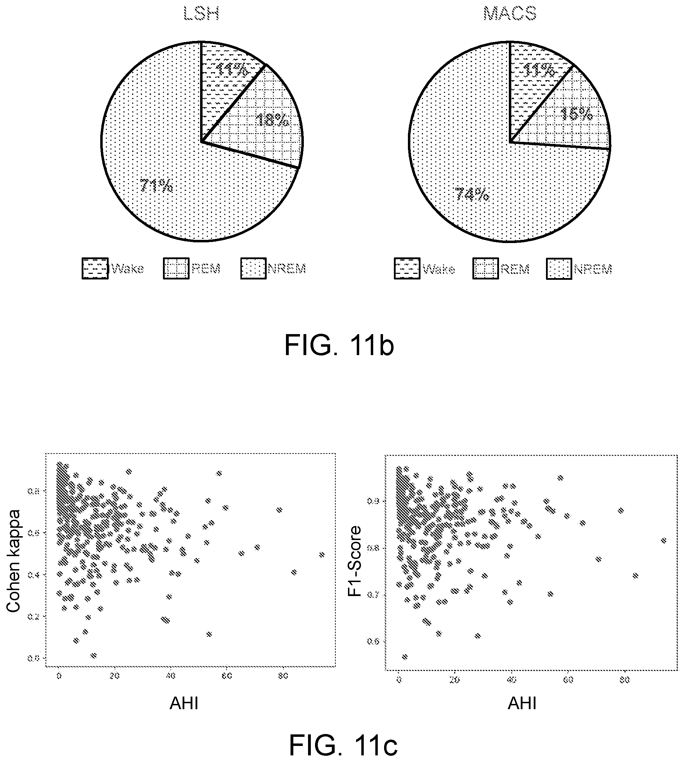

FIG. 11b shows the distribution of sleep stages amongst the test sets for the First Dataset and the Second Dataset, according to the manual scorings. The distribution of the test sets is similar to the distribution of the training sets, except that the distribution of sleep stages for the Second Dataset is now more similar to the First Dataset test set, that is wake is the least common sleep stage in both cases.

### 6.2 - Results: Precision, Recall, F1 score, Accuracy

To evaluate the performance of the classifier the following metrics were used $Precision = \frac{{t}_{p}}{{t}_{p} + {f}_{p}}$ $Recall = \frac{{t}_{p}}{{t}_{p} + {f}_{n}}$ ${F}_{1} = 2 ⋅ \frac{precision ⋅ recall}{precision + recall}$ $Accuracy = \frac{{t}_{p} + {t}_{n}}{{t}_{p} + {t}_{n} + {f}_{p} + {f}_{n}}$ Where t_p= number of true positives, f_p= number of false positives, and f_n= number of false negatives.

Tables 6.3-6.8 show the precision, recall and F1-score (classification report), as well as confusion matrix, for the cross-validation set and test set of the First Dataset, both for the final combined model, but also separately for the two models (the dense one and the GRU one). The cross-validated confusion matrix is the sum of the confusion matrices of each of the five folds. Then to calculate the cross-validated classification report, the combined confusion matrix is used to calculate t_p, f_p and f_n, which is then used to calculate the precision, recall and F 1-score.

**Table 6.3 The cross-validated results for the combined GRU model and Dense model on the First Dataset. Classification report (showing Precision, Recall, and F1-score) on the left, confusion matrix on the right.**

| | Precision | Recall | F1-score | Accuracy | Support |
|---|---|---|---|---|---|
| Wake | 0.77 | 0.69 | 0.73 | 0.69 | 17065 |
| REM | 0.85 | 0.81 | 0.83 | 0.81 | 25889 |
| NREM | 0.91 | 0.94 | 0.92 | 0.94 | 100959 |
| Avg/Total | 0.88 | 0.89 | 0.88 | 0.89 | 143913 |

| | Predicted | | | |
|---|---|---|---|---|
| | | Wake | REM | NREM |
| Manual | Wake | 11740 | 447 | 4878 |
| | REM | 421 | 20973 | 4495 |
| | NREM | 3036 | 3131 | 94792 |

**Table 6.4 The cross-validated results for only the GRU model (no prediction for the ends) on the First Dataset. Classification report (showing Precision, Recall, and F1-score) on the left, confusion matrix on the right.**

| | Precision | Recall | F1-score | Accuracy | Support |
|---|---|---|---|---|---|
| Wake | 0.75 | 0.68 | 0.71 | 0.68 | 14623 |
| REM | 0.86 | 0.81 | 0.83 | 0.81 | 25812 |
| NREM | 0.91 | 0.94 | 0.93 | 0.94 | 99902 |
| Avg/Total | 0.89 | 0.89 | 0.89 | 0.89 | 140337 |

| | Predicted | | | | |
|---|---|---|---|---|---|
| Manual | | Wake | REM | NREM | |
| | Wake | 9896 | 423 | 4304 | |
| | REM | 411 | 20930 | 4471 | |
| | NREM | 2847 | 3105 | 93950 | |

**Table 6.5 The cross-validated results for only the Dense model applied to the ends of each epoch on the First Dataset. Classification report (showing Precision, Recall, and F1-score) on the left, confusion matrix on the right.**

| | Precision | Recall | F1-score | Accuracy | Support |
|---|---|---|---|---|---|
| Wake | 0.9 | 0.76 | 0.82 | 0.76 | 2442 |
| REM | 0.46 | 0.56 | 0.51 | 0.56 | 77 |
| NREM | 0.58 | 0.8 | 0.67 | 0.8 | 1057 |
| Avg/Total | 0.8 | 0.76 | 0.77 | 0.76 | 3576 |

| | Predicted | | | |
|---|---|---|---|---|
| | | Wake | REM | NREM |
| Manual | Wake | 1844 | 24 | 574 |
| | REM | 10 | 43 | 24 |
| | NREM | 189 | 26 | 842 |

**Table 6.6 The test set results for the combined GRU model and Dense model on the First Dataset. Classification report (showing Precision, Recall, and F1-score) on the left, confusion matrix on the right.**

| | Precision | Recall | F1-score | Accuracy | Support |
|---|---|---|---|---|---|
| Wake | 0.79 | 0.64 | 0.71 | 0.64 | 3063 |
| REM | 0.89 | 0.78 | 0.83 | 0.78 | 4509 |
| NREM | 0.9 | 0.95 | 0.93 | 0.95 | 17493 |
| Avg/Total | 0.88 | 0.89 | 0.88 | 0.89 | 25065 |

| | Predicted | | | |
|---|---|---|---|---|
| | | Wake | REM | NREM |
| Manual | Wake | 1972 | 58 | 1033 |
| | REM | 117 | 3519 | 873 |
| | NREM | 401 | 389 | 16703 |

**Table 6.7 The test set results for only the GRU model (no prediction for the ends) on the First Dataset. Classification report (showing Precision, Recall, and F1-score) on the left, confusion matrix on the right.**

| | Precision | Recall | F1-score | Accuracy | Support |
|---|---|---|---|---|---|
| Wake | 0.78 | 0.63 | 0.7 | 0.63 | 2629 |
| REM | 0.89 | 0.78 | 0.83 | 0.78 | 4466 |
| NREM | 0.9 | 0.96 | 0.93 | 0.96 | 17322 |
| Avg/Total | 0.89 | 0.89 | 0.89 | 0.89 | 24417 |

| | Predicted | | | |
|---|---|---|---|---|
| | | Wake | REM | NREM |
| Manual | Wake | 1666 | 46 | 917 |
| | REM | 100 | 3503 | 863 |
| | NREM | 371 | 387 | 16564 |

**Table 6.8 The test set results for only the Dense model applied to the ends of each epoch on the First Dataset. Classification report (showing Precision, Recall, and F1-score) on the left, confusion matrix on the right.**

| | Precision | Recall | F1-score | Accuracy | Support |
|---|---|---|---|---|---|
| Wake | 0.87 | 0.71 | 0.78 | 0.71 | 434 |
| REM | 0.53 | 0.37 | 0.44 | 0.37 | 43 |
| NREM | 0.52 | 0.81 | 0.64 | 0.81 | 171 |
| Avg/Total | 0.75 | 0.71 | 0.72 | 0.71 | 648 |

| | Predicted | | | |
|---|---|---|---|---|
| | | Wake | REM | NREM |
| Manual | Wake | 306 | 12 | 116 |
| | REM | 17 | 16 | 10 |
| | NREM | 30 | 2 | 139 |

### 6.3 - Results: AHI

Apnea-Hypopnea Index (AHI) is a metric that is used to indicate the severity of sleep apnea and is measured by counting the apneas over the night and dividing by total sleep time. When AHI is calculated, all manually labelled apneas that occur during wakes may be ignored. Therefore, it is helpful to validate whether using PG+ for sleep staging results in a more accurate AHI. As a reference the estimated sleep is used, which identifies periods where the patient is upright as wake. The AHI is then calculated for these three sleep scorings with the manual labelled sleep stages as the targets and the estimated sleep scoring as a lower limit. To validate the AHI values from these scorings the AHI values are divided into classes based on literature and the metrics introduced in the section above are calculated.

The F1 score is then calculated for the AHI based on the following four classes:
1. AHI < 5
2.$5 \leq AHI < 15$
3. $15 \leq AHI < 30$
4. $30 \leq AHI$
as well as the following 3 classes:
1. AHI < 5
2. $5 \leq AHI < 15$
3. $15 \leq AHI .$

Tables 6.9-6.13 below show the precision, recall, F1-score, and confusion matrix of the AHI for the cross-validation set and test set of the First Dataset. Results are reported both for the final combined model, but also separately for the two models (the dense one and the GRU one).

**Table 6.9 The cross-validated results of the four-class AHI categorization on the First Dataset, using the PG+ method. At the top is the classification report, at the bottom is the confusion matrix.**

| | Precision | Recall | F1 Score | Support |
|---|---|---|---|---|
| AHI<5 | 0.98 | 1 | 0.99 | 123 |
| 5≤AHI<15 | 1 | 0.88 | 0.94 | 17 |
| 15≤AHI<30 | 1 | 1 | 1 | 6 |
| 30 ≤AHI | 1 | 1 | 1 | 3 |
| AVG / Total | 0.99 | 0.99 | 0.99 | 149 |

| | Predicted | | | | |
|---|---|---|---|---|---|
| | | AHI<5 | 5≤AHI<15 | 15≤AHI<3 | 30≤AHI |
| | AHI<5 | 123 | 0 | 0 | 0 |
| Manual | 5≤AHI<15 | 2 | 15 | 0 | 0 |
| | 15≤AHI<30 | 0 | 0 | 6 | 0 |
| | 30≤AHI | 0 | 0 | 0 | 3 |

**Table 6.10 The cross-validated results of the four-class AHI categorization on the First Dataset, estimating sleep based on position and activity. At the top is the classification report, at the bottom is the confusion matrix.**

| | Precision | Recall | F1 Score | Support |
|---|---|---|---|---|
| AHI<5 | 0.99 | 0.99 | 0.99 | 123 |
| 5≤AHI<15 | 0.94 | 0.94 | 0.94 | 17 |
| 15≤AHI<30 | 0.75 | 1 | 0.86 | 6 |
| 30 ≤AHI | 1 | 0.33 | 0.5 | 3 |
| AVG / Total | 0.98 | 0.97 | 0.97 | 149 |

| | Predicted | | | | |
|---|---|---|---|---|---|
| | | AHI<5 | 5≤AHI<15 | 15≤AHI<3 | 30≤AHI |
| | AHI<5 | 122 | 1 | 0 | 0 |
| Manual | 5≤AHI<15 | 1 | 16 | 0 | 0 |
| | 15≤AHI<30 | 0 | 0 | 6 | 0 |
| | 30≤AHI | 0 | 0 | 2 | 1 |

**Table 6.11 The cross-validated results of the four-class AHI categorization on the First Dataset, estimating sleep the whole night. At the top is the classification report, at the bottom is the confusion matrix.**

| | Precision | Recall | F1 Score | Support |
|---|---|---|---|---|
| AHI<5 | 0.99 | 0.99 | 0.99 | 123 |
| 5≤AHI<15 | 0.89 | 0.94 | 0.91 | 17 |
| 15≤AHI<30 | 0.71 | 0.83 | 0.77 | 6 |
| 30 ≤AHI | 1 | 0.33 | 0.5 | 3 |
| AVG / Total | 0.98 | 0.97 | 0.97 | 149 |

| | Predicted | | | | |
|---|---|---|---|---|---|
| | | AHI<5 | 5≤AHI<15 | 15≤AHI<3 | 30≤AHI |
| | AHI<5 | 122 | 1 | 0 | 0 |
| Manual | 5≤AHI<15 | 1 | 16 | 0 | 0 |
| | 15≤AHI<30 | 0 | 1 | 5 | 0 |
| | 30≤AHI | 0 | 0 | 2 | 1 |

**Table 6.12 The cross-validated results of the three-class AHI categorization on the First Dataset, using the PG+ method. At the top is the classification report, at the bottom is the confusion matrix.**

| | Precision | Recall | F1 Score | Support |
|---|---|---|---|---|
| AHI<5 | 0.98 | 1 | 0.99 | 123 |
| 5≤AHI<15 | 1 | 0.88 | 0.94 | 17 |
| 15 ≤AHI | 1 | 1 | 1 | 9 |
| AVG / Total | 0.99 | 0.99 | 0.99 | 149 |

| | Predicted | | | |
|---|---|---|---|---|
| | | AHI<5 | 5≤AHI<15 | 15≤AHI |
| Manual | AHI<5 | 123 | 0 | 0 |
| | 5≤AHI<15 | 2 | 15 | 0 |
| | 15 ≤AHI | 0 | 0 | 9 |

**Table 6.13 The cross-validated results of the three-class AHI categorization on the First Dataset, estimating sleep based on position and activity. At the top is the classification report, at the bottom is the confusion matrix.**

| | Precision | Recall | F1 Score | Support |
|---|---|---|---|---|
| AHI<5 | 0.99 | 0.99 | 0.99 | 123 |
| 5≤AHI<15 | 0.94 | 0.94 | 0.94 | 17 |
| 15 ≤AHI | 1 | 1 | 1 | 9 |
| AVG / Total | 0.99 | 0.99 | 0.99 | 149 |

| | Predicted | | | |
|---|---|---|---|---|
| | | AHI<5 | 5≤AHI<15 | 15≤AHI |
| Manual | AHI<5 | 122 | 1 | 0 |
| | 5≤AHI<15 | 1 | 16 | 0 |
| | 15 ≤AHI | 0 | 0 | 9 |

**Table 6.14 The cross-validated results of the three-class AHI categorization on the First Dataset, estimating sleep the whole night. At the top is the classification report, at the bottom is the confusion matrix.**

| | Precision | Recall | F1 Score | Support |
|---|---|---|---|---|
| AHI<5 | 0.99 | 0.99 | 0.99 | 123 |
| 5≤AHI<15 | 0.89 | 0.94 | 0.91 | 17 |
| 15 ≤AHI | 1 | 0.89 | 0.94 | 9 |
| AVG / Total | 0.98 | 0.98 | 0.98 | 149 |

| | Predicted | | | |
|---|---|---|---|---|
| | | AHI<5 | 5≤AHI<15 | 15≤AHI |
| Manual | AHI<5 | 122 | 1 | 0 |
| | 5≤AHI<15 | 1 | 16 | 0 |
| | 15 ≤AHI | 0 | 1 | 8 |

### 6.5 - Results: Cohen's Kappa

Cohen's Kappa is a common metric for quantifying the interrater agreement between human scorers. For scoring sleep-stages, the Cohen's Kappa lies between 0.61 and 0.80.

The cross-validated Cohen's Kappa score for our method was 0.75 for the final model (which includes both recurrent neural network and a dense neural network for the ends).

The Cohen's Kappa score for the test set was 0.74 for the same model.

### 6.6 - Results: F1 and Cohen's Kappa compared to AHI

To investigate the influence of AHI on the performance of the scorer, the F1 score is looked at and Cohen's kappa of individual recordings. The combined datasets (First Dataset and Second Dataset) are used and the model trained on the features extracted by the research team. The reason for using the combined datasets is to get more even AHI distribution, as the First Dataset has in general lower AHI values, while the Second Dataset has higher AHI values.

FIG. 11c shows the distribution of F1-score (to the right) and Cohen's Kappa (to the left) of the individual recordings for the combined datasets (First Dataset and Second Dataset), in total 338 recordings.

Furthermore, the AHI of the recording is plotted against the F1-score and the Cohen's Kappa, showing that AHI does not seem to affect theses scores.

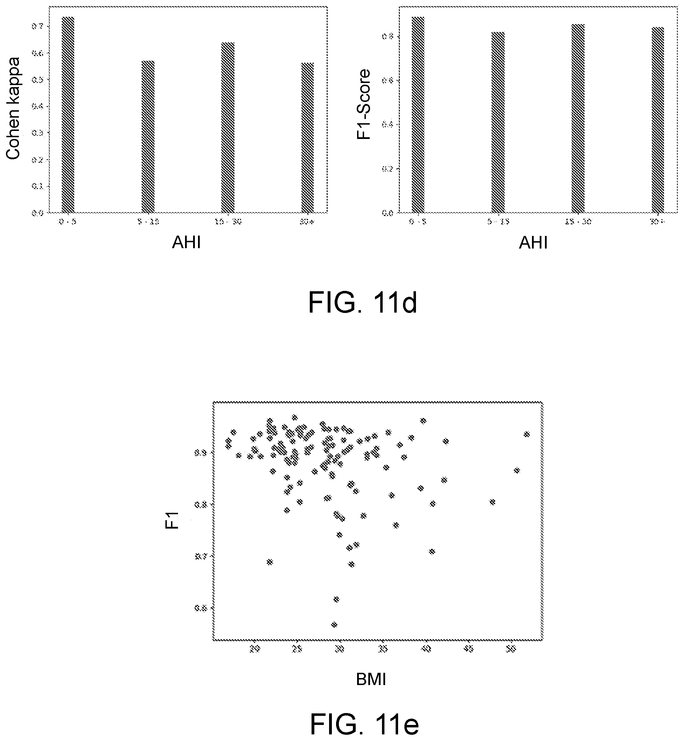

FIG. 11d shows the average F1-score (to the right) and average Cohen's Kappa (to the left) of recordings within each of the AHI categories: 0-5, 5-15, 15-30, and above 30. The first category is the largest, with 145 recordings, the next one has 87 recordings, the third one has 64 recordings, and the last one has 42 recordings. There is little difference of average F1-score and average Cohen's Kappa between these classes, although a bit more difference between classes for the Cohen's Kappa.

### 6.7 - Results: F1 compared to BMI and gender

On the First Dataset, BMI and gender information are provided. FIG. 11e shows the distribution of F1-score compared to BMI for the entire First Dataset, excluding the recordings with BMI = 0.0 or BMI > 150 (wrongly documented BMI), in total 125 recordings. It can be seen that there is no apparent trend between F1-score and BMI.Note that the BMI comparison is not possible for the Second Dataset, as the BMI values are seldomly reported.

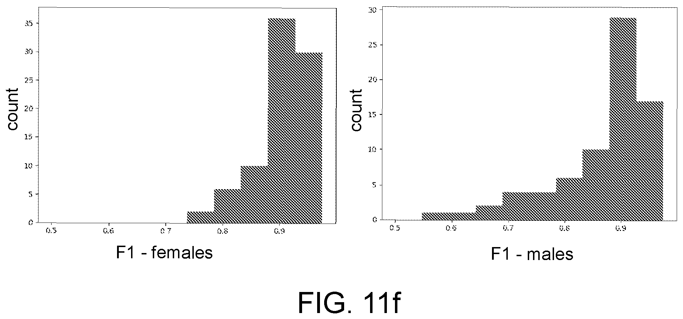

FIG. 11f shows the distribution of F1-score of females (to the left) and males (to the right) for the entire First Dataset, in total 158 recordings. It can be seen that there is little trend between F1-score and gender, even though males seem to have more outliers than females. Note that the gender comparison is not possible for the Second Dataset, as the Second Dataset only includes of males.

### 7 - Appendix

In this chapter the main changes from previous version of the PG+ sleep scorer are discussed. Further, the main things tried that did not improve the classifier are discussed.

### 7.1 - Changes from Previous Version

Some changes have been done to the previous version of PG+, both regarding the feature extraction and model architecture.

### 7.1.1 - Feature Extraction

As has been mentioned above, many features were cut from the previous version. A total of 45 features that used the ECG were removed since the ECG is not always included in a PG sleep study. These features include both the heart rate variability and the cardiorespiratory interaction features. Dropping these features did not have any effect on the performance. The 8 features using the oximeter signal to count pulse-wave amplitude drops were removed since the oximeter signal has been found to be unreliable. The next features to be removed were the network graph, the detrended fluctuation analysis and the coefficient of variance features. These features were deemed too costly in implementation and the latter two groups were also calculation heavy. The last round of removed features was removed by request of the software team and included various respiratory features which were calculated over more than one epoch. A list of removed respiratory features can be found below. In total 98 features were removed, leaving only 61 features for the model. The performance was only minimally affected by this, with the drop in cross-validated F1-score being within 1%.

**Table 7.1 List of removed respiratory features**

| | | |
|---|---|---|
| BandPowerAbdomenVol 0.05-0.5 Hz | NoxPhaseMean | RRv-VG Mean Degree |
| BandPowerAbdomenVol 0.05-10 Hz | NoxPhaseStd | RRv-VG STD Degree |
| BandPowerAbdomenVol 1-10 Hz | AbdomenThoraxVolPhase | RRv-VG # of Small Degree |
| BandPowerAbdomenFlow 0.05-0.5 Hz | AbdomenThoraxFlowPhase | RRv-VG # Large Degree |
| BandPowerAbdomenFlow 0.05-10 Hz | AbdomenRIPVolPhase | RRv-DVG Mean Degree |
| BandPowerAbdomenFlow 1-10 Hz | AbdomenRIPFlowPhase | RRv-DVG STD Degree |
| BandPowerThoraxVol 0.05-0.5 Hz | ThoraxRIPVolPhase | RRv-DVG # of Small Degree |
| BandPowerThoraxVol 0.05-10 Hz | ThoraxRIPFlowPhase | RRv-DVG # Large Degree |
| BandPowerThoraxVol 1-10 Hz | | RRv-VG Assortativity Coefficient |
| BandPowerThoraxFlow 0.05-0.5 Hz | | RRv-DVG Assortativity Coefficient |
| BandPowerThoraxFlow 0.05-10 Hz | | RRv-VG Degree Distb Slope |
| BandPowerThoraxFlow 1-10 Hz | | RRv-DVG Degree Distb slope |
| BandPowerRIPVol 0.05-0.5 Hz | | RRv-DFA Alpha |
| BandPowerRIPVol 0.05-10 Hz | | RRv-Coefficient of Variance |
| BandPowerRIPVol 1-10 Hz | | |

Some minor errors were fixed in the features that were left but these fixes did not result in an increase in performance. The biggest changes were done to the skewness features, which had been incorrectly implemented, although the idea behind them was correct. The time constant features required the inhalation start of the breaths to be at zero, which was not the case, so that had to be accounted for. The way the breaths detected by the breath-by-breath algorithm were distributed between epochs was also changed. In the previous version the breaths were split between the epochs by their peak index, but this was changed to the end index. This was done by request from the software team since it was better for the breath data to include some data points from the past rather than the future.

The feature extraction code was also refactored to reduce running time. Some calculations were made more efficient, with the biggest change in running time being in the function that splits the breaths between the epochs.

All in all, the reduction and fixing of features did not lead to a significant change in performance. The extraction time was not checked for every change, but the average extraction time for each recording was reduced from around 10 minutes down to around 10 seconds after all the unwanted features had been dropped and the code optimized.

### 7.1.2 - Classifier

The classifier was simplified to a single neural network, with both dense layers and a recurrent layer, whereas the previous classifier was composed of two separate neural networks (a dense one and a recurrent one). Further, early stopping was introduced to minimize training time and to help reduce overfitting. Learning rate was also changed from being static to dynamic, so it is reduced on plateau. Some other hyper-parameters were also changed, such as the dropout rate and the timesteps for the recurrent network. The new model was easier to tune and gave a higher cross-validated F1-score.

### 7.2 - Things Tried

Different things were tried by the inventors with regard to both feature extraction and classifier architecture, that did not in this study noticeably improve performance.

### 7.2.1 Feature Extraction

Apart from removing unwanted features, a few minor changes and fixes were made to various features. Many of these changes did not result in an increase of performance, but it was decided to keep them to have the features correctly calculated.

Some new features relating to the zero-flow ratio of the RIP inductance signals were calculated. These features were supposed to help the classifier with detecting the REM stage but did not improve the performance. These features were therefore not included.

### 7.2.2 - Classifier

First, some variations of the structure of the original classifier were tried, including:
1. Skipping the dense pretrained model, keeping the same structure for the RNN model;
2. Use bidirectional LSTM model, instead of a GRU model;
3. Change patience in "Reduce learning rate on plateau", tried values between 2-10;
4. Tried timesteps=10, future=5;
5. Cascaded binary classifier, where first classify sleep-wake, then classify sleep into nrem/rem.
   a. Tried two versions of rem-nrem classification, first using weights=0, performed badly
   b. Second training only on rem-nrem, performed better than the first version;
6. Ensembling models trained with different seeds and different features;
7. tanh activation instead of relu in the dense net, for a balanced input to the RNN;
8. Try leaky-relu activation instead of relu, in the dense net.

As used herein, RNN is Recurrent Neural Network a type of an artificial neural network which learns patterns which occur over time. An example of where RNNs are used is in language processing where the order and context of letters or words is of importance.

LSTM is Long-Short Term Memory a type of an artificial neural network which learns patterns which occur over time. The LSTM is a different type of an artificial neural network than RNN which both are designed to learn temporal patterns.

GRU is Gated Recurrent Unit, a building block of RNN artificial neural networks.

Secondly, some variations of the structure of the current classifier described in chapter 5.1 were tried.
1. The dense layer preceding GRU has 50 nodes instead of 70;
2. The GRU layer with 70 nodes instead of 50;
3. Using dropout values from 0.20-0.50 (dropout of 0.22 performed best).

There are endless alternative neural network structures that would yield a comparative or similar result. There are even neural network structures, such as Convolutional Neural Networks (CNN), which could use the raw recorded signals without having to have the features extracted or predetermined as we do here.

The number of layers, number of units, the connection between layers, the types of layers (RNN, LSTM, Dense, CNN, etc), activation functions, and other parameters can all be changed without reducing the performance of the model. Therefore, this disclosure should be not limited to a particular number of layers, number of units, the connection between layers, the types of layers (RNN, LSTM, Dense, CNN, etc.), activation functions, or other parameters that can be changed without reducing the performance of the model

During the development several parameters and characteristics were tested to optimize performance. Section 7.2.2 above mentions several variations used when selecting the best performing solution. Each variation impacts the performance slightly, although the changes in performance may be changes in the time needed to train the model, the time it takes to have the model make a prediction, or the performance of the model predicting a correct sleep stage.

Although the subject matter of this disclosure has been described in language specific to structural features and/or methodological acts, it is to be understood that the subject matter defined in the appended claims is not necessarily limited to the described features or acts described above, or the order of the acts described above. Rather, the described features and acts are disclosed as example forms of implementing the claims.

Embodiments of the present disclosure may comprise or utilize a special-purpose or general-purpose computer system that includes computer hardware, such as, for example, one or more processors and system memory, as discussed in greater detail below. Embodiments within the scope of the present disclosure also include physical and other computer-readable media for carrying or storing computer-executable instructions and/or data structures. Such computer-readable media can be any available media that can be accessed by a general-purpose or special-purpose computer system. Computer-readable media that store computer-executable instructions and/or data structures are computer storage media. Computer-readable media that carry computer-executable instructions and/or data structures are transmission media. Thus, by way of example, embodiments of the disclosure can comprise at least two distinctly different kinds of computer-readable media: computer storage media and transmission media.

Computer storage media are physical storage media that store computer-executable instructions and/or data structures. Physical storage media include computer hardware, such as RAM, ROM, EEPROM, solid state drives ("SSDs"), flash memory, phase-change memory ("PCM"), optical disk storage, magnetic disk storage or other magnetic storage devices, or any other hardware storage device(s) which can be used to store program code in the form of computer-executable instructions or data structures, which can be accessed and executed by a general-purpose or special-purpose computer system to implement the disclosed functionality of the disclosure.

Transmission media can include a network and/or data links which can be used to carry program code in the form of computer-executable instructions or data structures, and which can be accessed by a general-purpose or special-purpose computer system. A "network" may be defined as one or more data links that enable the transport of electronic data between computer systems and/or modules and/or other electronic devices. When information is transferred or provided over a network or another communications connection (either hardwired, wireless, or a combination of hardwired or wireless) to a computer system, the computer system may view the connection as transmission media. Combinations of the above should also be included within the scope of computer-readable media.

Further, upon reaching various computer system components, program code in the form of computer-executable instructions or data structures can be transferred automatically from transmission media to computer storage media (or vice versa). For example, computer-executable instructions or data structures received over a network or data link can be buffered in RAM within a network interface module (e.g., a "NIC"), and then eventually transferred to computer system RAM and/or to less volatile computer storage media at a computer system. Thus, it should be understood that computer storage media can be included in computer system components that also (or even primarily) utilize transmission media.

Computer-executable instructions may comprise, for example, instructions and data which, when executed by one or more processors, cause a general-purpose computer system, special-purpose computer system, or special-purpose processing device to perform a certain function or group of functions. Computer-executable instructions may be, for example, binaries, intermediate format instructions such as assembly language, or even source code.

The disclosure of the present application may be practiced in network computing environments with many types of computer system configurations, including, but not limited to, personal computers, desktop computers, laptop computers, message processors, hand-held devices, multi-processor systems, microprocessor-based or programmable consumer electronics, network PCs, minicomputers, mainframe computers, mobile telephones, PDAs, tablets, pagers, routers, switches, and the like. The disclosure may also be practiced in distributed system environments where local and remote computer systems, which are linked (either by hardwired data links, wireless data links, or by a combination of hardwired and wireless data links) through a network, both perform tasks. As such, in a distributed system environment, a computer system may include a plurality of constituent computer systems. In a distributed system environment, program modules may be located in both local and remote memory storage devices.

The disclosure of the present application may also be practiced in a cloud-computing environment. Cloud computing environments may be distributed, although this is not required. When distributed, cloud computing environments may be distributed internationally within an organization and/or have components possessed across multiple organizations. In this description and the following claims, "cloud computing" is defined as a model for enabling on-demand network access to a shared pool of configurable computing resources (e.g., networks, servers, storage, applications, and services). The definition of "cloud computing" is not limited to any of the other numerous advantages that can be obtained from such a model when properly deployed.

A cloud-computing model can be composed of various characteristics, such as on-demand self-service, broad network access, resource pooling, rapid elasticity, measured service, and so forth. A cloud-computing model may also come in the form of various service models such as, for example, Software as a Service ("SaaS"), Platform as a Service ("PaaS"), and Infrastructure as a Service ("IaaS"). The cloud-computing model may also be deployed using different deployment models such as private cloud, community cloud, public cloud, hybrid cloud, and so forth.

Some embodiments, such as a cloud-computing environment, may comprise a system that includes one or more hosts that are each capable of running one or more virtual machines. During operation, virtual machines emulate an operational computing system, supporting an operating system and perhaps one or more other applications as well. In some embodiments, each host includes a hypervisor that emulates virtual resources for the virtual machines using physical resources that are abstracted from view of the virtual machines. The hypervisor also provides proper isolation between the virtual machines. Thus, from the perspective of any given virtual machine, the hypervisor provides the illusion that the virtual machine is interfacing with a physical resource, even though the virtual machine only interfaces with the appearance (e.g., a virtual resource) of a physical resource. Examples of physical resources including processing capacity, memory, disk space, network bandwidth, media drives, and so forth.

Certain terms are used throughout the description and claims to refer to particular methods, features, or components. As those having ordinary skill in the art will appreciate, different persons may refer to the same methods, features, or components by different names. This disclosure does not intend to distinguish between methods, features, or components that differ in name but not function. The figures are not necessarily drawn to scale. Certain features and components herein may be shown in exaggerated scale or in somewhat schematic form and some details of conventional elements may not be shown or described in interest of clarity and conciseness.

Although various example embodiments have been described in detail herein, many modifications are possible in the example embodiments without materially departing from the concepts of present disclosure. Accordingly, any such modifications are intended to be included in the scope of this disclosure. Likewise, while the disclosure herein contains many specifics, these specifics should not be construed as limiting the scope of the disclosure or of any of the appended claims, but merely as providing information pertinent to one or more specific embodiments that may fall within the scope of the disclosure and the appended claims. Any described features from the various embodiments disclosed may be employed in combination. In addition, other embodiments of the present disclosure may also be devised which lie within the scopes of the disclosure and the appended claims. Each addition, deletion, and modification to the embodiments that falls within the meaning and scope of the claims is to be embraced by the claims.

Certain embodiments and features may have been described using a set of numerical upper limits and a set of numerical lower limits. It should be appreciated that ranges including the combination of any two values, e.g., the combination of any lower value with any upper value, the combination of any two lower values, and/or the combination of any two upper values are contemplated unless otherwise indicated. Certain lower limits, upper limits and ranges may appear in one or more claims below. Any numerical value is "about" or "approximately" the indicated value, and takes into account experimental error and variations that would be expected by a person having ordinary skill in the art.

## Claims

1. A method for a determining a sleep stage of a subject, the method comprising:
obtaining by a processor (38) one or more respiratory signals, the one or more respiratory signals being an indicator of a respiratory activity of the subject, the one or more respiratory signals includes a thoracic respiratory inductance plethysmography (RIP) signal and an abdomen respiratory inductance plethysmography (RIP) signal;
deriving one or more respiratory parameters from the thoracic respiratory inductance plethysmography (RIP) signal and the abdomen respiratory inductance plethysmography (RIP) signal, including a delay between the thoracic respiratory inductance plethysmography (RIP) signal and the abdomen respiratory inductance plethysmography (RIP) signal,
extracting by the processor one or more features from the thoracic respiratory inductance plethysmography (RIP) signal, the abdomen respiratory inductance plethysmography (RIP) signal, and the derived respiratory parameters,
determining by the processor a sleep stage of the subject based on the one or more extracted features by performing a classification of the extracted features based on a classifier, wherein the classifier is a neural network, decision tree or trees, forests of decision trees, clustering, and/or a support vector machine.

2. The method according to claim 1, wherein the first respiratory component signal includes the sum of the abdomen and thorax respiratory volume signals (RIPSum), a time derivative of the abdomen respiratory volume signal, a time derivative of the thorax respiratory volume signal, or a time derivative of the sum of the abdomen respiratory volume signal and the thorax respiratory volume signal (RIPflow).

3. The method according to claim 1, wherein the classifier is a neural network.

4. The method according to claim 3, wherein the neural network is a recurrent neural network or convolutional neural network (CNN).

5. The method according to claim 1, further including deriving one or more respiratory parameters from the thoracic respiratory inductance plethysmography (RIP) signal and the abdomen respiratory inductance plethysmography (RIP) signal, wherein the one or more respiratory parameters derived from the thoracic respiratory inductance plethysmography (RIP) signal and the abdomen respiratory inductance plethysmography (RIP) signal is used in the computer-based classification.

6. The method according to claim 1, wherein extracting features from the thoracic respiratory inductance plethysmography (RIP) signal and the abdomen respiratory inductance plethysmography (RIP) signal includes extracting a feature related to respiratory rate, a first harmonic, a DC component, a breath-by-breath characteristics, breath amplitude, breath length, a zero- flow ratio, an activity feature, an activity feature derived from the accelerometer signal, RIP phase, skewness of breaths, max flow in, max flow out, a ratio of max flow in and max flow out, a time constant of inhalation and/or exhaustion, or mean and standard deviations, of difference mean ratios thereof.

7. The method according to any of the preceding the claims, further comprising pre-processing of the one or more respiratory signals before extracting features from the thoracic respiratory inductance plethysmography (RIP) signal and the abdomen respiratory inductance plethysmography (RIP) signal.

8. A system for determining sleep stage of a subject, the system comprising:
a receiver configured to receive one or more respiratory signals, the one or more respiratory signals being an indicator of a respiratory activity of the subject, the one or more respiratory signals including a thoracic respiratory inductance plethysmography (RIP) signal and an abdomen respiratory inductance plethysmography (RIP) signal;
a processor (38) configured to:
derive one or more respiratory parameters from the thoracic respiratory inductance plethysmography (RIP) signal and the abdomen respiratory inductance plethysmography (RIP) signal, including a delay between the thoracic respiratory inductance plethysmography (RIP) signal and the abdomen respiratory inductance plethysmography (RIP) signal, and
extract one or more features from the thoracic respiratory inductance plethysmography (RIP) signal, the abdomen respiratory inductance plethysmography (RIP) signal and the derived respiratory parameters,
wherein the processor is further configured to determine a sleep stage of the subject based on the one or more extracted features using a classifier, wherein the classifier is a neural network, decision tree or trees, forests of decision trees, clustering, and/or a support vector machine.

9. The system according to claim 8, wherein the first respiratory component signal further includes a sum of the abdomen and thorax respiratory volume signals (RIPSum), a time derivative of the abdomen respiratory volume signal, a time derivative of the thorax respiratory volume signal, or a time derivative of the sum of the abdomen respiratory volume signal and the thorax respiratory volume signal (RIPflow).

10. The system according to claim 8, wherein the classifier is a neural network.

11. The system according to claim 10, wherein the neural network is a recurrent neural network or convolutional neural network (CNN).

12. The system according to claim 8, where the processor (38) is configured to derive one or more respiratory parameters from the thoracic respiratory inductance plethysmography (RIP) signal and the abdomen respiratory inductance plethysmography (RIP) signal, wherein the one or more respiratory parameters derived from the thoracic respiratory inductance plethysmography (RIP) signal and the abdomen respiratory inductance plethysmography (RIP) signal is used in the computer-based classification.

13. The system according to claim 9, where the processor (38) extracting the features from the thoracic respiratory inductance plethysmography (RIP) signal and the abdomen respiratory inductance plethysmography (RIP) signal includes extracting a feature related to respiratory rate, a first harmonic, a DC component, a breath-by-breath characteristics, breath amplitude, breath length, a zero-flow ratio, an activity feature, an activity feature derived from the accelerometer signal, RIP phase, skewness of breaths, max flow in, max flow out, a ratio of max flow in and max flow out, a time constant of inhalation and/or exhaustion, or mean and standard deviations, of difference mean ratios thereof.

14. A hardware storage device having stored thereon computer executable instructions which, when executed by one or more processors (38) of a computer system, configure the computer system to perform at least the following:
receive, from a receiver, one or more respiratory signals, including obtaining a thoracic respiratory inductance plethysmography (RIP) signal and obtaining an abdomen respiratory inductance plethysmography (RIP) signal, the one or more respiratory signals being an indicator of a respiratory activity of the subject;
derive one or more respiratory parameters from the thoracic respiratory inductance plethysmography (RIP) signal and the abdomen respiratory inductance plethysmography (RIP) signal, including a delay between the thoracic respiratory inductance plethysmography (RIP) signal and the abdomen respiratory inductance plethysmography (RIP) signal,
extract features from the thoracic respiratory inductance plethysmography (RIP) signal, the abdomen respiratory inductance plethysmography (RIP) signal and the derived respiratory parameters,
wherein the instructions when executed by one or more processors (28) of a computer system, configure the computer system to determine a sleep stage of the subject based on the one or more extracted features using a classifier, wherein the classifier is a neural network, decision tree or trees, forests of decision trees, clustering, and/or a support vector machine.

## Patentansprüche

1. Verfahren zum Bestimmen einer Schlafphase eines Subjekts, wobei das Verfahren Folgendes umfasst:
Erfassen eines oder mehrerer Atemsignale durch einen Prozessor (38), wobei das eine oder die mehreren Atemsignale ein Indikator einer Atemtätigkeit des Subjekts sind, wobei das eine oder die mehreren Atemsignale ein Thorax-Ateminduktionsplethysmografie-(respiratory inductance plethysmography - RIP-) Signal und ein Abdomen-Ateminduktionsplethysmografie- (RIP-) Signal einschließen,
Ableiten eines oder mehrerer Atmungsparameter aus dem Thorax-Ateminduktionsplethysmografie- (RIP-) Signal und dem Abdomen-Ateminduktionsplethysmografie- (RIP-) Signal, einschließlich einer Verzögerung zwischen dem Thorax-Ateminduktionsplethysmografie- (RIP-) Signal und dem Abdomen-Ateminduktionsplethysmografie- (RIP-) Signal,
Extrahieren eines oder mehrerer Merkmale aus dem Thorax-Ateminduktionsplethysmografie- (RIP-) Signal, dem Abdomen-Ateminduktionsplethysmografie- (RIP-) Signal und den abgeleiteten Atmungsparametern durch den Prozessor,
Bestimmen durch den Prozessor einer Schlafphase des Subjekts auf Grundlage des einen oder der mehreren extrahierten Merkmale durch Durchführen einer Klassifizierung der extrahierten Merkmale auf Grundlage eines Klassifikators, wobei der Klassifikator ein neuronales Netz, Entscheidungsbaum oder -bäume, Wälder von Entscheidungsbäumen, Clustering und/oder eine Stützvektormaschine ist.

2. Verfahren nach Anspruch 1, wobei das erste Atemkomponentensignal die Summe des Abdomen- und des Thorax-Atemvolumensignals (RIPSum), eine zeitliche Ableitung des Abdomen-Atemvolumensignals, eine zeitliche Ableitung des Thorax-Atemvolumensignals oder eine zeitliche Ableitung der Summe des Abdomen-Atemvolumensignals und des Thorax-Atemvolumensignals (RIPflow) einschließt.

3. Verfahren nach Anspruch 1, wobei der Klassifikator ein neuronales Netz ist.

4. Verfahren nach Anspruch 3, wobei das neuronale Netz ein rekurrentes neuronales Netz oder ein neuronales Faltungsnetz (convolutional neural network - CNN) ist.

5. Verfahren nach Anspruch 1, das ferner das Ableiten eines oder mehrerer Atmungsparameter aus dem Thorax-Ateminduktionsplethysmografie- (RIP-) Signal und dem Abdomen-Ateminduktionsplethysmografie- (RIP-) Signal einschließt, wobei der eine oder die mehreren Atmungsparameter, die aus dem Thorax-Ateminduktionsplethysmografie- (RIP-) Signal und dem Abdomen-Ateminduktionsplethysmografie- (RIP-) Signal abgeleitet werden, bei der rechnergestützten Klassifizierung verwendet werden.

6. Verfahren nach Anspruch 1, wobei das Extrahieren von Merkmalen aus dem Thorax-Ateminduktionsplethysmografie- (RIP-) Signal und dem Abdomen-Ateminduktionsplethysmografie- (RIP-) Signal das Extrahieren eines Merkmals einschließt, das mit Atemfrequenz, einer ersten Harmonischen, einer GS-Komponente, eine Charakteristik Atemzug für Atemzug, Atemzugamplitude, Atemzuglänge, einem Nullfluss-Verhältnis, einem Aktivitätsmerkmal, einem Aktivitätsmerkmal, das von dem Akzelerometersignal abgeleitet ist, RIP-Phase, Asymmetrie von Atemzügen, maximaler Einströmung, maximaler Ausströmung, einem Verhältnis von maximaler Einströmung und maximaler Ausströmung, einer Zeitkonstanten von Einatmung und/oder Ausatmung oder mittlerer und Standardabweichung von Differenzmittelverhältnissen derselben in Beziehung steht.

7. Verfahren nach einem der vorhergehenden Ansprüche, das ferner das Vorverarbeiten des einen oder der mehreren Atemsignale vor dem Extrahieren von Merkmalen aus dem Thorax-Ateminduktionsplethysmografie- (RIP-) Signal und dem Abdomen-Ateminduktionsplethysmografie- (RIP-) Signal umfasst.

8. System zum Bestimmen einer Schlafphase eines Subjekts, wobei das System Folgendes umfasst:
einen Empfänger, der dafür konfiguriert ist, ein oder mehrere Atemsignale zu empfangen, wobei das eine oder die mehreren Atemsignale ein Indikator einer Atemtätigkeit des Subjekts sind, wobei das eine oder die mehreren Atemsignale ein Thorax-Ateminduktionsplethysmografie- (RIP-) Signal und ein Abdomen-Ateminduktionsplethysmografie- (RIP-) Signal einschließen,
einen Prozessor (38), der zu Folgendem konfiguriert ist:
einen oder mehrere Atmungsparameter aus dem Thorax-Ateminduktionsplethysmografie- (RIP-) Signal und dem Abdomen-Ateminduktionsplethysmografie- (RIP-) Signal abzuleiten, einschließlich einer Verzögerung zwischen dem Thorax-Ateminduktionsplethysmografie- (RIP-) Signal und dem Abdomen-Ateminduktionsplethysmografie- (RIP-) Signal,
ein oder mehrere Merkmale aus dem Thorax-Ateminduktionsplethysmografie- (RIP-) Signal, dem Abdomen-Ateminduktionsplethysmografie- (RIP-) Signal und den abgeleiteten Atmungsparametern zu extrahieren,
wobei der Prozessor ferner dafür konfiguriert ist, eine Schlafphase des Subjekts auf Grundlage des einen oder der mehreren extrahierten Merkmale unter Verwendung eines Klassifikators zu bestimmen, wobei der Klassifikator ein neuronales Netz, Entscheidungsbaum oder -bäume, Wälder von Entscheidungsbäumen, Clustering und/oder eine Stützvektormaschine ist.

9. System nach Anspruch 8, wobei das erste Atemkomponentensignal ferner eine Summe des Abdomen- und des Thorax-Atemvolumensignals (RIPSum), eine zeitliche Ableitung des Abdomen-Atemvolumensignals, eine zeitliche Ableitung des Thorax-Atemvolumensignals oder eine zeitliche Ableitung der Summe des Abdomen-Atemvolumensignals und des Thorax-Atemvolumensignals (RIPflow) einschließt.

10. System nach Anspruch 8, wobei der Klassifikator ein neuronales Netz ist.

11. System nach Anspruch 10, wobei das neuronale Netz ein rekurrentes neuronales Netz oder ein neuronales Faltungsnetz (CNN) ist.

12. System nach Anspruch 8, wobei der Prozessor (38) dafür konfiguriert ist, einen oder mehrere Atmungsparameter aus dem Thorax-Ateminduktionsplethysmografie- (RIP-) Signal und dem Abdomen-Ateminduktionsplethysmografie-(RIP-) Signal abzuleiten, wobei der eine oder die mehreren Atmungsparameter, die aus dem Thorax-Ateminduktionsplethysmografie- (RIP-) Signal und dem Abdomen-Ateminduktionsplethysmografie- (RIP-) Signal abgeleitet werden, bei der rechnergestützten Klassifizierung verwendet werden.

13. System nach Anspruch 9, wobei, dass der Prozessor (38) die Merkmalen aus dem Thorax-Ateminduktionsplethysmografie- (RIP-) Signal und dem Abdomen-Ateminduktionsplethysmografie- (RIP-) Signal extrahiert, das Extrahieren eines Merkmals einschließt, das mit Atemfrequenz, einer ersten Harmonischen, einer GS-Komponente, eine Charakteristik Atemzug für Atemzug, Atemzugamplitude, Atemzuglänge, einem Nullfluss-Verhältnis, einem Aktivitätsmerkmal, einem Aktivitätsmerkmal, das von dem Akzelerometersignal abgeleitet ist, RIP-Phase, Asymmetrie von Atemzügen, maximaler Einströmung, maximaler Ausströmung, einem Verhältnis von maximaler Einströmung und maximaler Ausströmung, einer Zeitkonstanten von Einatmung und/oder Ausatmung oder mittlerer und Standardabweichung von Differenzmittelverhältnissen derselben in Beziehung steht.

14. Hardware-Speichervorrichtung, die auf derselben gespeicherte rechnerausführbare Anweisungen aufweist, die, wenn sie durch einen oder mehrere Prozessoren (38) eines Rechnersystems ausgeführt werden, das Rechnersystem dafür konfigurieren, zumindest das Folgende durchzuführen:
von einem Empfänger ein oder mehrere Atemsignale zu empfangen, was das Erhalten eines Thorax-Ateminduktionsplethysmografie- (RIP-) Signals und eines Abdomen-Ateminduktionsplethysmografie- (RIP-) Signals einschließt, wobei das eine oder die mehreren Atemsignale ein Indikator einer Atemtätigkeit des Subjekts sind,
einen oder mehrere Atmungsparameter aus dem Thorax-Ateminduktionsplethysmografie- (RIP-) Signal und dem Abdomen-Ateminduktionsplethysmografie- (RIP-) Signal abzuleiten, einschließlich einer Verzögerung zwischen dem Thorax-Ateminduktionsplethysmografie- (RIP-) Signal und dem Abdomen-Ateminduktionsplethysmografie-(RIP-) Signal,
Merkmale aus dem Thorax-Ateminduktionsplethysmografie-(RIP-) Signal, dem Abdomen-Ateminduktionsplethysmografie- (RIP-) Signal und den abgeleiteten Atmungsparametern zu extrahieren,
wobei die Anweisungen, wenn sie durch einen oder mehrere Prozessoren (38) eines Rechnersystems ausgeführt werden, das Rechnersystem dafür konfigurieren, eine Schlafphase des Subjekts auf Grundlage des einen oder der mehreren extrahierten Merkmale unter Verwendung eines Klassifikators zu bestimmen, wobei der Klassifikator ein neuronales Netz, Entscheidungsbaum oder -bäume, Wälder von Entscheidungsbäumen, Clustering und/oder eine Stützvektormaschine ist.

## Revendications

1. Méthode pour une détermination d'une phase de sommeil d'un sujet, méthode comprenant :
obtention par un processeur (38) d'un ou plusieurs signaux respiratoires, l'un ou les plusieurs signaux respiratoires étant un indicateur d'une activité respiratoire du sujet, l'un ou les plusieurs signaux respiratoires incluent un signal de pléthysmographie d'inductance respiratoire (RIP) thoracique et un signal de pléthysmographie d'inductance respiratoire (RIP) abdominale ;
déduction d'un ou plusieurs paramètres respiratoires du signal de pléthysmographie d'inductance respiratoire (RIP) thoracique et du signal de pléthysmographie d'inductance respiratoire (RIP) abdominale, incluant un délai entre le signal de pléthysmographie d'inductance respiratoire (RIP) thoracique et le signal de pléthysmographie d'inductance respiratoire (RIP) abdominale,
extraction par le processeur d'une ou plusieurs caractéristiques du signal de pléthysmographie d'inductance respiratoire (RIP) thoracique, du signal de pléthysmographie d'inductance respiratoire (RIP) abdominale et des paramètres respiratoires déduits,
détermination par le processeur d'une phase de sommeil du sujet sur la base de l'une ou des plusieurs caractéristiques extraites en effectuant une classification des caractéristiques extraites sur la base d'un classificateur, dans laquelle le classificateur est un réseau neuronal, un ou des arbres décisionnels, des forêts d'arbres décisionnels, un regroupement et/ou une machine à vecteurs de support.

2. Méthode selon la revendication 1, dans laquelle le signal de la première composante respiratoire inclut la somme des signaux de volume respiratoire abdominal et thoracique (RIPSum), une dérivée temporelle du signal de volume respiratoire abdominal, une dérivée temporelle du signal de volume respiratoire thoracique ou une dérivée temporelle de la somme du signal de volume respiratoire abdominal et du signal de volume respiratoire thoracique (RIPflow).

3. Méthode selon la revendication 1, dans laquelle le classificateur est un réseau neuronal.

4. Méthode selon la revendication 3, dans laquelle le réseau neuronal est un réseau neuronal récurrent ou un réseau neuronal convolutif (CNN).

5. Méthode selon la revendication 1, incluant en outre la déduction d'un ou plusieurs paramètres respiratoires du signal de pléthysmographie d'inductance respiratoire (RIP) thoracique et du signal de pléthysmographie d'inductance respiratoire (RIP) abdominale, dans laquelle l'un ou les plusieurs paramètres respiratoires déduits du signal de pléthysmographie d'inductance respiratoire (RIP) thoracique et du signal de pléthysmographie d'inductance respiratoire (RIP) abdominale sont utilisés dans la classification informatisée.

6. Méthode selon la revendication 1, dans laquelle l'extraction de caractéristiques du signal de pléthysmographie d'inductance respiratoire (RIP) thoracique et du signal de pléthysmographie d'inductance respiratoire (RIP) abdominale inclut l'extraction d'une caractéristique liée à la fréquence respiratoire, d'une première valeur harmonique, d'une composante continue, d'une caractéristique respiration par respiration, d'une amplitude de respiration, d'une longueur de respiration, d'un rapport de débit nul, d'une caractéristique d'activité, d'une caractéristique d'activité déduite du signal d'accéléromètre, d'une phase de RIP, d'une asymétrie des respirations, d'un débit maximal entrant, d'un débit maximal sortant, d'un rapport entre débit maximal entrant et débit maximal sortant, d'une constante temporelle d'inspiration et/ou d'expiration ou d'écarts moyens et d'écarts-types, rapports moyens d'une différence correspondants.

7. Méthode selon l'une des revendications précédentes, comprenant en outre un prétraitement de l'un ou des plusieurs signaux respiratoires avant d'extraire des caractéristiques du signal de pléthysmographie d'inductance respiratoire (RIP) thoracique et du signal de pléthysmographie d'inductance respiratoire (RIP) abdominale.

8. Système pour déterminer une phase de sommeil d'un sujet, système comprenant :
un récepteur configuré pour recevoir un ou plusieurs signaux respiratoires, l'un ou les plusieurs signaux respiratoires étant un indicateur d'une activité respiratoire du sujet, l'un ou les plusieurs signaux respiratoires incluant un signal de pléthysmographie d'inductance respiratoire (RIP) thoracique et un signal de pléthysmographie d'inductance respiratoire (RIP) abdominale ;
un processeur (38) configuré pour :
déduire un ou plusieurs paramètres respiratoires du signal de pléthysmographie d'inductance respiratoire (RIP) thoracique et du signal de pléthysmographie d'inductance respiratoire (RIP) abdominale, incluant un délai entre le signal de pléthysmographie d'inductance respiratoire (RIP) thoracique et le signal de pléthysmographie d'inductance respiratoire (RIP) abdominale, et
extraire une ou plusieurs caractéristiques du signal de pléthysmographie d'inductance respiratoire (RIP) thoracique, du signal de pléthysmographie d'inductance respiratoire (RIP) abdominale et des paramètres respiratoires déduits,
dans lequel le processeur est en outre configuré pour déterminer une phase de sommeil du sujet sur la base de l'une ou des plusieurs caractéristiques extraites en utilisant un classificateur, dans lequel le classificateur est un réseau neuronal, un ou des arbres décisionnels, des forêts d'arbres décisionnels, un regroupement et/ou une machine à vecteurs de support.

9. Système selon la revendication 8, dans lequel le signal de la première composante respiratoire inclut en outre une somme des signaux de volume respiratoire abdominal et thoracique (RIPSum), une dérivée temporelle du signal de volume respiratoire abdominal, une dérivée temporelle du signal de volume respiratoire thoracique ou une dérivée temporelle de la somme du signal de volume respiratoire abdominal et du signal de volume respiratoire thoracique (RIPflow).

10. Système selon la revendication 8, dans lequel le classificateur est un réseau neuronal.

11. Système selon la revendication 10, dans lequel le réseau neuronal est un réseau neuronal récurrent ou un réseau neuronal convolutif (CNN).

12. Système selon la revendication 8, dans lequel le processeur (38) est configuré pour déduire un ou plusieurs paramètres respiratoires du signal de pléthysmographie d'inductance respiratoire (RIP) thoracique et du signal de pléthysmographie d'inductance respiratoire (RIP) abdominale, dans lequel l'un ou les plusieurs paramètres respiratoires déduits du signal de pléthysmographie d'inductance respiratoire (RIP) thoracique et du signal de pléthysmographie d'inductance respiratoire (RIP) abdominale sont utilisés dans la classification informatisée.

13. Système selon la revendication 9, dans lequel l'extraction par le processeur (38) des caractéristiques du signal de pléthysmographie d'inductance respiratoire (RIP) thoracique et du signal de pléthysmographie d'inductance respiratoire (RIP) abdominale inclut l'extraction d'une caractéristique liée à la fréquence respiratoire, d'une première valeur harmonique, d'une composante continue, d'une caractéristique respiration par respiration, d'une amplitude de respiration, d'une longueur de respiration, d'un rapport de débit nul, d'une caractéristique d'activité, d'une caractéristique d'activité déduite du signal d'accéléromètre, d'une phase de RIP, d'une asymétrie des respirations, d'un débit maximal entrant, d'un débit maximal sortant, d'un rapport entre débit maximal entrant et débit maximal sortant, d'une constante temporelle d'inspiration et/ou d'expiration ou d'écarts moyens et d'écarts-types, rapports moyens d'une différence correspondants.

14. Dispositif de stockage matériel sur lequel sont stockées des instructions exécutables par un ordinateur qui, lorsqu'elles sont exécutées par un ou plusieurs processeurs (38) d'un système informatique, configurent le système informatique pour qu'il effectue au moins les opérations suivantes :
recevoir d'un récepteur un ou plusieurs signaux respiratoires, incluant l'obtention d'un signal de pléthysmographie d'inductance respiratoire (RIP) thoracique et l'obtention d'un signal de pléthysmographie d'inductance respiratoire (RIP abdominale, l'un ou les plusieurs signaux respiratoires étant un indicateur d'une activité respiratoire du sujet ;
déduire un ou plusieurs paramètres respiratoires du signal de pléthysmographie d'inductance respiratoire (RIP) thoracique et du signal de pléthysmographie d'inductance respiratoire (RIP) abdominale, incluant un délai entre le signal de pléthysmographie d'inductance respiratoire (RIP) thoracique et le signal de pléthysmographie d'inductance respiratoire (RIP) abdominale,
extraire des caractéristiques du signal de pléthysmographie d'inductance respiratoire (RIP) thoracique, du signal de pléthysmographie d'inductance respiratoire (RIP) abdominale et des paramètres respiratoires déduits,
dans lequel les instructions, lorsqu'elles sont exécutées par un ou plusieurs processeurs (38) d'un système informatique, configurent le système informatique pour qu'il détermine une phase de sommeil du sujet sur la base de l'une ou des plusieurs caractéristiques extraites en utilisant un classificateur, dans lequel le classificateur est un réseau neuronal, un ou des arbres décisionnels, des forêts d'arbres décisionnels, un regroupement et/ou une machine à vecteurs de support.
